# EUROPEAN PATENT SPECIFICATION

(11) **EP 3 288 560 B1**
(45) Date of publication and mention of the grant of the patent: **07.10.2020**
(21) Application number: 16783980.2
(22) Date of filing: 22.04.2016
(51) Int. Cl.: C07J 41/00, C07J 9/00

(54) **METHODS FOR THE SYNTHESIS OF CERAGENINS**
VERFAHREN ZUR SYNTHESE VON CERAGENINEN
PROCÉDÉS DE SYNTHÈSE DES CÉRAGÉNINES

(30) Priority: 22.04.2015 US 201562151026 P; 21.05.2015 US 201562165027 P; 13.07.2015 US 201562191926 P
(43) Date of publication of application: 07.03.2018
(73) Proprietor: Brigham Young University, Provo, UT 84602 (US)
(72) Inventor: SAVAGE, Paul B., Mapleton, UT 84664 (US); RANDALL, Jared Lynn, Smyrna, New York 13464 (US)
(74) Representative: Schwarz & Partner Patentanwälte OG
(86) International application number: PCT/US2016/028947
(87) International publication number: WO 2016/172553

(56) References cited:
- WO-A1-2009/049370
- WO-A1-2016/186821
- WO-A2-2013/165574
- US-A1- 2014 194 401
- US-B1- 8 557 031
- US-B2- 8 211 879

## Description

### BACKGROUND OF THE INVENTION

### 1. Field

The present application relates to the fields of pharmaceutical chemistry, biochemistry, and medicine. In particular, the present application relates to methods of making cationic steroidal antimicrobials ("CSAs" or "ceragenins").

### 2. Related Technology

Endogenous antimicrobial peptides, such as the human cathelicidin LL-37, play key roles in innate immunity. LL-37 is found in airway mucus and is believed to be important in controlling bacterial growth in the lung. Antimicrobial peptides are found in organisms ranging from mammals to amphibians to insects to plants. The ubiquity of antimicrobial peptides has been used as evidence that these compounds do not readily engender bacterial resistance. In addition, considering the varied sequences of antimicrobial peptides among diverse organisms, it is apparent that they have evolved independently multiple times. Thus, antimicrobial peptides appear to be one of "Nature's" primary means of controlling bacterial growth. However, clinical use of antimicrobial peptides presents significant issues including the relatively high cost of producing peptide-based therapeutics, the susceptibility of peptides to proteases generated by the host and by bacterial pathogens, and deactivation of antimicrobial peptides by proteins and DNA in lung mucosa.

An attractive means of harnessing the antibacterial activities of antimicrobial peptides without the issues delineated above is to develop non-peptide mimics of antimicrobial peptides that display the same broad-spectrum antibacterial activity utilizing the same mechanism of action. Non-peptide mimics would offer lower-cost synthesis and potentially increased stability to proteolytic degradation. In addition, control of water solubility and charge density may be used to control association with proteins and DNA in lung mucosa.

With over 1,600 examples of antimicrobial peptides known, it is possible to categorize the structural features common to them. While the primary sequences of these peptides vary substantially, morphologies adopted by a vast majority are similar. Those that adopt alpha helix conformations juxtapose hydrophobic side chains on one face of the helix with cationic (positively charged) side chains on the opposite side. As similar morphology is found in antimicrobial peptides that form beta sheet structures: hydrophobic side chains on one face of the sheet and cationic side chains on the other.

We have developed small molecule, non-peptide mimics of antimicrobial peptides, termed ceragenins or CSAs. These compounds reproduce the amphiphilic morphology in antimicrobial peptides, represented above by CSA-13, and display potent, as well as diverse, biological activities (including, but not limited to anti-bacterial, anti-cancer, antiinflammatory, promoting bone growth, promoting wound healing, etc.). Lead ceragenins can be produced at a large scale, and because they are not peptide based, they are not substrates for proteases. Consequently, the ceragenins represented an attractive compound class for producing pharmaceutically-relevant treatments.

US-A-8,211,879 discloses a process for the production of ceragenins by reducing cholic acid to the corresponding bile alcohol, followed by etherification of the OH groups and then amination of free OH groups (both on the ether groups and the C₁₇-side chain).

### SUMMARY

Certain embodiments described herein relate to methods of making a compound of Formula (I) or Formula (II), comprising the steps of:
(a) reacting a compound of Formula (1) and R₁R₂-NH 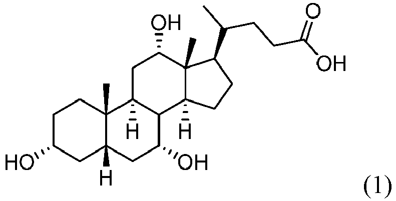 to form a compound of Formula (2): 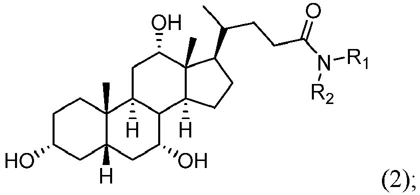
(b) reacting a compound of Formula (2) with a compound of Formula (A), 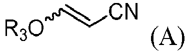 in the presence of acid and a phase transfer catalyst, to form a compound of Formula (3): 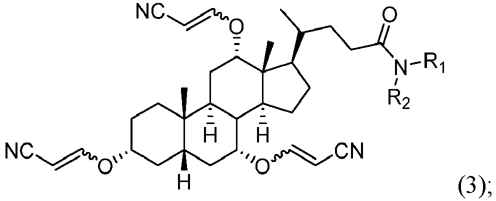 and
(c) subjecting a compound of Formula (3) to reducing conditions to form a compound of Formula (I): 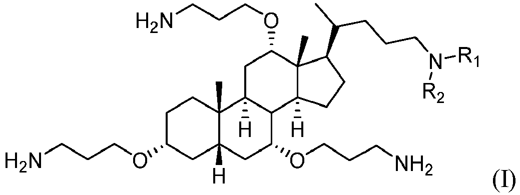 or a compound of Formula (II): 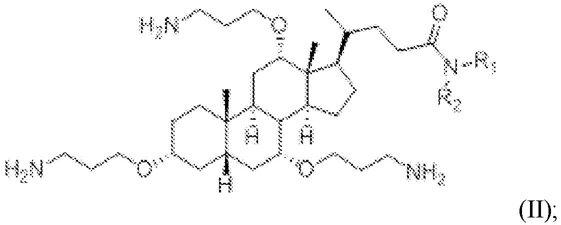 wherein:
   R₁ and R₂ are independently selected from the group consisting of hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, optionally substituted amido, and a suitable amine protecting group; and
   R₃ is selected from the group consisting of optionally substituted C₁-C₁₄ alkyl, optionally substituted C₂-C₂₄ alkenyl, optionally substituted C₂-C₂₄ alkynyl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, and optionally substituted (5 to 10 membered heterocyclyl)-C₁-C₆ alkyl.

When producing the compounds of formula (II), the method is carried out at a temperature of at least 0°C, at least 10°C, at least 20°C, at least 30°C, at least 40°C, at least 50°C, at least 60°C, at least 70°C, at least 80°C, at least 90°C, at least 100°C, at least 110°C, at least 120°C, at least 130°C, at least 140°C, at least 150°C, at least 160°C, at least 170°C, or at least 180°C.

Advantanges of the CSA compounds disclosed herein include, but are not limited to, comparable and/or improved antimicrobial activity, stability, and/or pharmaceutical administerability compared to existing CSA compounds and/or simplified synthetis of final CSA compounds and/or intermediate CSA compounds compared to existing synthetic routes.

Additional features and advantages will be set forth in part in the description that follows, and in part will be obvious from the description, or may be learned by practice of the embodiments disclosed herein. It is to be understood that both the foregoing brief summary and the following detailed description are exemplary and explanatory only and are not restrictive of the embodiments disclosed herein or as claimed.

### DETAILED DESCRIPTION

### Definitions:

Unless defined otherwise, all technical and scientific terms used herein have the same meaning as is commonly understood by one of ordinary skill in the art. In the event that there are a plurality of definitions for a term herein, those in this section prevail unless stated otherwise. As used in the specification and the appended claims, the singular forms "a," "an" and "the" include plural referents unless the context clearly dictates otherwise. Unless otherwise indicated, conventional methods of mass spectroscopy, NMR, HPLC, protein chemistry, biochemistry, recombinant DNA techniques and pharmacology are employed. The use of "or" or "and" means "and/or" unless stated otherwise. Furthermore, use of the term "including" as well as other forms, such as "include", "includes," and "included," is not limiting. As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least." When used in the context of a process, the term "comprising" means that the process includes at least the recited steps, but may include additional steps. When used in the context of a compound, composition, or device, the term "comprising" means that the compound, composition, or device includes at least the recited features or components, but may also include additional features or components.

The section headings used herein are for organizational purposes only and are not to be construed as limiting the subject matter described.

As used herein, "Cₐ to C_{b}" or "C_{a-b}" in which "a" and "b" are integers refer to the number of carbon atoms in the specified group. That is, the group can contain from "a" to "b", inclusive, carbon atoms. Thus, for example, a "C₁ to C₄ alkyl" or "C₁₋₄ alkyl" group refers to all alkyl groups having from 1 to 4 carbons, that is, CH₃-, CH₃CH₂-, CH₃CH₂CH₂-, (CH₃)₂CH-, CH₃CH₂CH₂CH₂-, CH₃CH₂CH(CH₃)- and (CH₃)₃C-.

The term "halogen" or "halo," as used herein, means any one of the radio-stable atoms of column 7 of the Periodic Table of the Elements, e.g., fluorine, chlorine, bromine, or iodine, with fluorine and chlorine being preferred.

As used herein, "alkyl" refers to a straight or branched hydrocarbon chain that is fully saturated (i.e., contains no double or triple bonds). The alkyl group may have 1 to 20 carbon atoms (whenever it appears herein, a numerical range such as "1 to 20" refers to each integer in the given range; *e.g.*, "1 to 20 carbon atoms" means that the alkyl group may consist of 1 carbon atom, 2 carbon atoms, 3 carbon atoms, *etc.*, up to and including 20 carbon atoms, although the present definition also covers the occurrence of the term "alkyl" where no numerical range is designated). The alkyl group may also be a medium size alkyl having 1 to 9 carbon atoms. The alkyl group could also be a lower alkyl having 1 to 4 carbon atoms. The alkyl group may be designated as "C₁₋₄ alkyl" or similar designations. By way of example only, "C₁₋₄ alkyl" indicates that there are one to four carbon atoms in the alkyl chain, i.e., the alkyl chain is selected from the group consisting of methyl, ethyl, propyl, iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl, hexyl, and the like.

As used herein, "alkoxy" refers to the formula -OR wherein R is an alkyl as is defined above, such as "C₁₋₉ alkoxy", including but not limited to methoxy, ethoxy, n-propoxy, 1-methylethoxy (isopropoxy), n-butoxy, iso-butoxy, sec-butoxy, and tert-butoxy, and the like.

As used herein, "alkylthio" refers to the formula -SR wherein R is an alkyl as is defined above, such as "C₁₋₉ alkylthio" and the like, including but not limited to methylmercapto, ethylmercapto, n-propylmercapto, 1-methylethylmercapto (isopropylmercapto), n-butylmercapto, iso-butylmercapto, sec-butylmercapto, tert-butylmercapto, and the like.

As used herein, "alkenyl" refers to a straight or branched hydrocarbon chain containing one or more double bonds. The alkenyl group may have 2 to 20 carbon atoms, although the present definition also covers the occurrence of the term "alkenyl" where no numerical range is designated. The alkenyl group may also be a medium size alkenyl having 2 to 9 carbon atoms. The alkenyl group could also be a lower alkenyl having 2 to 4 carbon atoms. The alkenyl group may be designated as "C₂₋₄ alkenyl" or similar designations. By way of example only, "C₂₋₄ alkenyl" indicates that there are two to four carbon atoms in the alkenyl chain, i.e., the alkenyl chain is selected from the group consisting of ethenyl, propen-1-yl, propen-2-yl, propen-3-yl, buten-1-yl, buten-2-yl, buten-3-yl, buten-4-yl, 1-methyl-propen-1-yl, 2-methyl-propen-1-yl, 1-ethyl-ethen-1-yl, 2-methyl-propen-3-yl, buta-1,3-dienyl, buta-1,2,-dienyl, and buta-1,2-dien-4-yl. Typical alkenyl groups include, but are in no way limited to, ethenyl, propenyl, butenyl, pentenyl, and hexenyl, and the like.

As used herein, "alkynyl" refers to a straight or branched hydrocarbon chain containing one or more triple bonds. The alkynyl group may have 2 to 20 carbon atoms, although the present definition also covers the occurrence of the term "alkynyl" where no numerical range is designated. The alkynyl group may also be a medium size alkynyl having 2 to 9 carbon atoms. The alkynyl group could also be a lower alkynyl having 2 to 4 carbon atoms. The alkynyl group may be designated as "C₂₋₄ alkynyl" or similar designations. By way of example only, "C₂₋₄ alkynyl" indicates that there are two to four carbon atoms in the alkynyl chain, i.e., the alkynyl chain is selected from the group consisting of ethynyl, propyn-1-yl, propyn-2-yl, butyn-1-yl, butyn-3-yl, butyn-4-yl, and 2-butynyl. Typical alkynyl groups include, but are in no way limited to, ethynyl, propynyl, butynyl, pentynyl, and hexynyl, and the like.

As used herein, "heteroalkyl" refers to a straight or branched hydrocarbon chain containing one or more heteroatoms, that is, an element other than carbon, including but not limited to, nitrogen, oxygen and sulfur, in the chain backbone. The heteroalkyl group may have 1 to 20 carbon atom, although the present definition also covers the occurrence of the term "heteroalkyl" where no numerical range is designated. The heteroalkyl group may also be a medium size heteroalkyl having 1 to 9 carbon atoms. The heteroalkyl group could also be a lower heteroalkyl having 1 to 4 carbon atoms. The heteroalkyl group may be designated as "C₁₋₄ heteroalkyl" or similar designations. The heteroalkyl group may contain one or more heteroatoms. By way of example only, "C₁₋₄ heteroalkyl" indicates that there are one to four carbon atoms in the heteroalkyl chain and additionally one or more heteroatoms in the backbone of the chain.

As used herein, "alkylene" means a branched, or straight chain fully saturated di-radical chemical group containing only carbon and hydrogen that is attached to the rest of the molecule via two points of attachment (i.e., an alkanediyl). The alkylene group may have 1 to 20 carbon atoms, although the present definition also covers the occurrence of the term alkylene where no numerical range is designated. The alkylene group may also be a medium size alkylene having 1 to 9 carbon atoms. The alkylene group could also be a lower alkylene having 1 to 4 carbon atoms. The alkylene group may be designated as "C₁₋₄ alkylene" or similar designations. By way of example only, "C₁₋₄ alkylene" indicates that there are one to four carbon atoms in the alkylene chain, i.e., the alkylene chain is selected from the group consisting of methylene, ethylene, ethan-1,1-diyl, propylene, propan-1,1-diyl, propan-2,2-diyl, 1-methyl-ethylene, butylene, butan-1,1-diyl, butan-2,2-diyl, 2-methyl-propan-1,1-diyl, 1-methyl-propylene, 2-methyl-propylene, 1,1-dimethyl-ethylene, 1,2-dimethyl-ethylene, and 1-ethyl-ethylene.

As used herein, "alkenylene" means a straight or branched chain di-radical chemical group containing only carbon and hydrogen and containing at least one carbon-carbon double bond that is attached to the rest of the molecule via two points of attachment. The alkenylene group may have 2 to 20 carbon atoms, although the present definition also covers the occurrence of the term alkenylene where no numerical range is designated. The alkenylene group may also be a medium size alkenylene having 2 to 9 carbon atoms. The alkenylene group could also be a lower alkenylene having 2 to 4 carbon atoms. The alkenylene group may be designated as "C₂₋₄ alkenylene" or similar designations. By way of example only, "C₂₋₄ alkenylene" indicates that there are two to four carbon atoms in the alkenylene chain, i.e., the alkenylene chain is selected from the group consisting of ethenylene, ethen-1,1-diyl, propenylene, propen-1,1-diyl, prop-2-en-1,1-diyl, 1-methyl-ethenylene, but-1-enylene, but-2-enylene, but-1,3-dienylene, buten-1,1-diyl, but-1,3-dien-1,1-diyl, but-2-en-1,1-diyl, but-3-en-1,1-diyl, 1-methyl-prop-2-en-1,1-diyl, 2-methyl-prop-2-en-1,1-diyl, 1-ethyl-ethenylene, 1,2-dimethyl-ethenylene, 1-methyl-propenylene, 2-methyl-propenylene, 3-methyl-propenylene, 2-methyl-propen-1,1-diyl, and 2,2-dimethyl-ethen-1,1-diyl.

The term "aromatic" refers to a ring or ring system having a conjugated pi electron system and includes both carbocyclic aromatic (e.g., phenyl) and heterocyclic aromatic groups (e.g., pyridine). The term includes monocyclic or fused-ring polycyclic (i.e., rings which share adjacent pairs of atoms) groups provided that the entire ring system is aromatic.

As used herein, "aryl" refers to an aromatic ring or ring system (i.e., two or more fused rings that share two adjacent carbon atoms) containing only carbon in the ring backbone. When the aryl is a ring system, every ring in the system is aromatic. The aryl group may have 6 to 18 carbon atoms, although the present definition also covers the occurrence of the term "aryl" where no numerical range is designated. In some embodiments, the aryl group has 6 to 10 carbon atoms. The aryl group may be designated as "C₆₋₁₀ aryl," "C₆ or C₁₀ aryl," or similar designations. Examples of aryl groups include, but are not limited to, phenyl, naphthyl, azulenyl, and anthracenyl.

As used herein, "aryloxy" and "arylthio" refers to RO- and RS-, in which R is an aryl as is defined above, such as "C₆₋₁₀ aryloxy" or "C₆₋₁₀ arylthio" and the like, including but not limited to phenyloxy.

An "aralkyl" or "arylalkyl" is an aryl group connected, as a substituent, via an alkylene group, such as "C₇₋₁₄ aralkyl" and the like, including but not limited to benzyl, 2-phenylethyl, 3-phenylpropyl, and naphthylalkyl. In some cases, the alkylene group is a lower alkylene group (i.e., a C₁₋₄ alkylene group).

As used herein, "heteroaryl" refers to an aromatic ring or ring system (i.e., two or more fused rings that share two adjacent atoms) that contain(s) one or more heteroatoms, that is, an element other than carbon, including but not limited to, nitrogen, oxygen and sulfur, in the ring backbone. When the heteroaryl is a ring system, every ring in the system is aromatic. The heteroaryl group may have 5-18 ring members (i.e., the number of atoms making up the ring backbone, including carbon atoms and heteroatoms), although the present definition also covers the occurrence of the term "heteroaryl" where no numerical range is designated. In some embodiments, the heteroaryl group has 5 to 10 ring members or 5 to 7 ring members. The heteroaryl group may be designated as "5-7 membered heteroaryl," "5-10 membered heteroaryl," or similar designations. Examples of heteroaryl rings include, but are not limited to, furyl, thienyl, phthalazinyl, pyrrolyl, oxazolyl, thiazolyl, imidazolyl, pyrazolyl, isoxazolyl, isothiazolyl, triazolyl, thiadiazolyl, pyridinyl, pyridazinyl, pyrimidinyl, pyrazinyl, triazinyl, quinolinyl, isoquinlinyl, benzimidazolyl, benzoxazolyl, benzothiazolyl, indolyl, isoindolyl, and benzothienyl.

A "heteroaralkyl" or "heteroarylalkyl" is heteroaryl group connected, as a substituent, via an alkylene group. Examples include but are not limited to 2-thienylmethyl, 3-thienylmethyl, furylmethyl, thienylethyl, pyrrolylalkyl, pyridylalkyl, isoxazollylalkyl, and imidazolylalkyl. In some cases, the alkylene group is a lower alkylene group (i.e., a C₁₋₄ alkylene group).

As used herein, "carbocyclyl" means a non-aromatic cyclic ring or ring system containing only carbon atoms in the ring system backbone. When the carbocyclyl is a ring system, two or more rings may be joined together in a fused, bridged or spiro-connected fashion. Carbocyclyls may have any degree of saturation provided that at least one ring in a ring system is not aromatic. Thus, carbocyclyls include cycloalkyls, cycloalkenyls, and cycloalkynyls. The carbocyclyl group may have 3 to 20 carbon atoms, although the present definition also covers the occurrence of the term "carbocyclyl" where no numerical range is designated. The carbocyclyl group may also be a medium size carbocyclyl having 3 to 10 carbon atoms. The carbocyclyl group could also be a carbocyclyl having 3 to 6 carbon atoms. The carbocyclyl group may be designated as "C₃₋₆ carbocyclyl" or similar designations. Examples of carbocyclyl rings include, but are not limited to, cyclopropyl, cyclobutyl, cyclopentyl, cyclohexyl, cyclohexenyl, 2,3-dihydro-indene, bicycle[2.2.2]octanyl, adamantyl, and spiro[4.4]nonanyl.

A "(carbocyclyl)alkyl" is a carbocyclyl group connected, as a substituent, via an alkylene group, such as "C₄₋₁₀ (carbocyclyl)alkyl" and the like, including but not limited to, cyclopropylmethyl, cyclobutylmethyl, cyclopropylethyl, cyclopropylbutyl, cyclobutylethyl, cyclopropylisopropyl, cyclopentylmethyl, cyclopentylethyl, cyclohexylmethyl, cyclohexylethyl, cycloheptylmethyl, and the like. In some cases, the alkylene group is a lower alkylene group.

As used herein, "cycloalkyl" means a fully saturated carbocyclyl ring or ring system. Examples include cyclopropyl, cyclobutyl, cyclopentyl, and cyclohexyl.

As used herein, "cycloalkenyl" means a carbocyclyl ring or ring system having at least one double bond, wherein no ring in the ring system is aromatic. An example is cyclohexenyl.

As used herein, "heterocyclyl" means a non-aromatic cyclic ring or ring system containing at least one heteroatom in the ring backbone. Heterocyclyls may be joined together in a fused, bridged or spiro-connected fashion. Heterocyclyls may have any degree of saturation provided that at least one ring in the ring system is not aromatic. The heteroatom(s) may be present in either a non-aromatic or aromatic ring in the ring system. The heterocyclyl group may have 3 to 20 ring members (i.e., the number of atoms making up the ring backbone, including carbon atoms and heteroatoms), although the present definition also covers the occurrence of the term "heterocyclyl" where no numerical range is designated. The heterocyclyl group may also be a medium size heterocyclyl having 3 to 10 ring members. The heterocyclyl group could also be a heterocyclyl having 3 to 6 ring members. The heterocyclyl group may be designated as "3-6 membered heterocyclyl" or similar designations. In preferred six membered monocyclic heterocyclyls, the heteroatom(s) are selected from one up to three of O, N or S, and in preferred five membered monocyclic heterocyclyls, the heteroatom(s) are selected from one or two heteroatoms selected from O, N, or S. Examples of heterocyclyl rings include, but are not limited to, azepinyl, acridinyl, carbazolyl, cinnolinyl, dioxolanyl, imidazolinyl, imidazolidinyl, morpholinyl, oxiranyl, oxepanyl, thiepanyl, piperidinyl, piperazinyl, dioxopiperazinyl, pyrrolidinyl, pyrrolidonyl, pyrrolidionyl, 4-piperidonyl, pyrazolinyl, pyrazolidinyl, 1,3-dioxinyl, 1,3-dioxanyl, 1,4-dioxinyl, 1,4-dioxanyl, 1,3-oxathianyl, 1,4-oxathiinyl, 1,4-oxathianyl, 2*H*-1,2-oxazinyl, trioxanyl, hexahydro-1,3,5-triazinyl, 1,3-dioxolyl, 1,3-dioxolanyl, 1,3-dithiolyl, 1,3-dithiolanyl, isoxazolinyl, isoxazolidinyl, oxazolinyl, oxazolidinyl, oxazolidinonyl, thiazolinyl, thiazolidinyl, 1,3-oxathiolanyl, indolinyl, isoindolinyl, tetrahydrofuranyl, tetrahydropyranyl, tetrahydrothiophenyl, tetrahydrothiopyranyl, tetrahydro-1,4-thiazinyl, thiamorpholinyl, dihydrobenzofuranyl, benzimidazolidinyl, and tetrahydroquinoline.

A "(heterocyclyl)alkyl" is a heterocyclyl group connected, as a substituent, via an alkylene group. Examples include, but are not limited to, imidazolinylmethyl and indolinylethyl.

As used herein, "acyl" refers to -C(=O)R, wherein R is hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein. Non-limiting examples include formyl, acetyl, propanoyl, benzoyl, and acryl.

An "O-carboxy" group refers to a "-OC(=O)R" group in which R is selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein.

A "C-carboxy" group refers to a "-C(=O)OR" group in which R is selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein. A non-limiting example includes carboxyl (i.e., -C(=O)OH).

A "cyano" group refers to a "-CN" group.

A "cyanato" group refers to an "-OCN" group.

An "isocyanato" group refers to a "-NCO" group.

A "thiocyanato" group refers to a "-SCN" group.

An "isothiocyanato" group refers to an " -NCS" group.

A "sulfinyl" group refers to an "-S(=O)R" group in which R is selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein.

A "sulfonyl" group refers to an "-SO₂R" group in which R is selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein.

An "S-sulfonamido" group refers to a "-SO₂NR_{A}R_{B}" group in which R_{A} and R_{B} are each independently selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein.

An "N-sulfonamido" group refers to a "-N(R_{A})SO₂R_{B}" group in which R_{A} and R_{b} are each independently selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein.

An "O-carbamyl" group refers to a "-OC(=O)NR_{A}R_{B}" group in which R_{A} and R_{B} are each independently selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein.

An "N-carbamyl" group refers to an "-N(R_{A})OC(=O)R_{B}" group in which R_{A} and R_{B} are each independently selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein.

An "O-thiocarbamyl" group refers to a "-OC(=S)NR_{A}R_{B}" group in which R_{A} and R_{B} are each independently selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein.

An "N-thiocarbamyl" group refers to an "-N(R_{A})OC(=S)R_{B}" group in which R_{A} and R_{B} are each independently selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein.

A "C-amido" group refers to a "-C(=O)NR_{A}R_{B}" group in which R_{A} and R_{B} are each independently selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, as defined herein.

An "N-amido" group refers to a "-N(R_{A})C(=O)R_{B}" group in which R_{A} and R_{B} are each independently selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, or optionally substituted (5 to 10 membered heterocyclyl)-C₁-C₆ alkyl, as defined herein.

An "amino" group refers to a "-NR_{A}R_{B}" group in which R_{A} and R_{B} are each independently selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, or a suitable amine protecting group, as defined herein. A non-limiting example includes free amino (i.e., -NH₂).

An "amide" or "amido" group refers to a "-NR-C(=O)-R group in which each R is independently selected from hydrogen, hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, or a suitable amine protecting group, as defined herein.

An "aminoalkyl" group refers to an amino group connected via an alkylene group.

An "alkoxyalkyl" group refers to an alkoxy group connected via an alkylene group, such as a "C₂₋₈ alkoxyalkyl" and the like.

As used herein, a substituted group is derived from the unsubstituted parent group in which there has been an exchange of one or more hydrogen atoms for another atom or group. Unless otherwise indicated, when a group is deemed to be "substituted," it is meant that the group is substituted with one or more substituents independently selected from C₁-C₁₄ alkyl, C₁-C₁₄ alkenyl, C₁-C₁₄ alkynyl, C₁-C₁₄ heteroalkyl, C₃-C₁₀ carbocyclyl (optionally substituted with halo, C₁-C₆ alkyl, C₁-C₆ alkoxy, C₁-C₆ haloalkyl, and C₁-C₆ haloalkoxy), C₃-C₁₀-carbocyclyl-C₁-C₆-alkyl (optionally substituted with halo, C₁-C₆ alkyl, C₁-C₆ alkoxy, C₁-C₆ haloalkyl, and C₁-C₆ haloalkoxy), 5-10 membered heterocyclyl (optionally substituted with halo, C₁-C₆ alkyl, C₁-C₆ alkoxy, C₁-C₆ haloalkyl, and C₁-C₆ haloalkoxy), 5-10 membered heterocyclyl-C₁-C₆-alkyl (optionally substituted with halo, C₁-C₆ alkyl, C₁-C₆ alkoxy, C₁-C₆ haloalkyl, and C₁-C₆ haloalkoxy), aryl (optionally substituted with halo, C₁-C₆ alkyl, C₁-C₆ alkoxy, C₁-C₆ haloalkyl, and C₁-C₆ haloalkoxy), aryl(C₁-C₆)alkyl (optionally substituted with halo, C₁-C₆ alkyl, C₁-C₆ alkoxy, C₁-C₆ haloalkyl, and C₁-C₆ haloalkoxy), 5-10 membered heteroaryl (optionally substituted with halo, C₁-C₆ alkyl, C₁-C₆ alkoxy, C₁-C₆ haloalkyl, and C₁-C₆ haloalkoxy), 5-10 membered heteroaryl(C₁-C₆)alkyl (optionally substituted with halo, C₁-C₆ alkyl, C₁-C₆ alkoxy, C₁-C₆ haloalkyl, and C₁-C₆ haloalkoxy), halo, cyano, hydroxy, C₁-C₆ alkoxy, C₁-C₆ alkoxy(C₁-C₆)alkyl (i.e., ether), aryloxy, sulfhydryl (mercapto), halo(C₁-C₆)alkyl (e.g., -CF₃), halo(C₁-C₆)alkoxy (e.g., -OCF₃), C₁-C₆ alkylthio, arylthio, amino, amino(C₁-C₆)alkyl, nitro, O-carbamyl, N-carbamyl, O-thiocarbamyl, N-thiocarbamyl, C-amido, N-amido, amido, S-sulfonamido, N-sulfonamido, C-carboxy, O-carboxy, acyl, cyanato, isocyanato, thiocyanato, isothiocyanato, sulfinyl, sulfonyl, and oxo (=O). Wherever a group is described as "optionally substituted" that group can be substituted with the above substituents.

It is to be understood that certain radical naming conventions can include either a mono-radical or a di-radical, depending on the context. For example, where a substituent requires two points of attachment to the rest of the molecule, it is understood that the substituent is a di-radical. For example, a substituent identified as alkyl that requires two points of attachment includes di-radicals such as -CH₂-, -CH₂CH₂-, -CH₂CH(CH₃)CH₂-, and the like. Other radical naming conventions clearly indicate that the radical is a di-radical such as "alkylene" or "alkenylene."

Wherever a substituent is depicted as a di-radical (*i.e.*, has two points of attachment to the rest of the molecule), it is to be understood that the substituent can be attached in any directional configuration unless otherwise indicated. Thus, for example, a substituent depicted as -AE- or 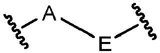 includes the substituent being oriented such that the A is attached at the leftmost attachment point of the molecule as well as the case in which A is attached at the rightmost attachment point of the molecule.

Where the compounds disclosed herein have at least one chiral center, they may exist as individual enantiomers and diastereomers or as mixtures of such isomers, including racemates. Separation of the individual isomers or selective synthesis of the individual isomers is accomplished by application of various methods which are well known to practitioners in the art. Unless otherwise indicated, all such isomers and mixtures thereof are included in the scope of the compounds disclosed herein. Furthermore, compounds disclosed herein may exist in one or more crystalline or amorphous forms. Unless otherwise indicated, all such forms are included in the scope of the compounds disclosed herein including any polymorphic forms. In addition, some of the compounds disclosed herein may form solvates with water (i.e., hydrates) or common organic solvents. Unless otherwise indicated, such solvates are included in the scope of the compounds disclosed herein.

The skilled artisan will recognize that some structures described herein may be resonance forms or tautomers of compounds that may be fairly represented by other chemical structures, even when kinetically; the artisan recognizes that such structures may only represent a very small portion of a sample of such compound(s). Such compounds are considered within the scope of the structures depicted, though such resonance forms or tautomers are not represented herein.

Isotopes may be present in the compounds described. Each chemical element as represented in a compound structure may include any isotope of said element. For example, in a compound structure a hydrogen atom may be explicitly disclosed or understood to be present in the compound. At any position of the compound that a hydrogen atom may be present, the hydrogen atom can be any isotope of hydrogen, including but not limited to hydrogen-1 (protium) and hydrogen-2 (deuterium). Thus, reference herein to a compound encompasses all potential isotopic forms unless the context clearly dictates otherwise.

Unless otherwise defined, all technical and scientific terms used herein have the same meaning as commonly understood by one of ordinary skill in the art to which these embodiments belong. The terminology used in the description herein is for describing particular embodiments only and is not intended to be limiting of the embodiments. As used in the specification and the appended claims, the singular forms "a," "an," and "the" are intended to include the plural forms as well, unless the context clearly indicates otherwise.

Terms and phrases used in this application, and variations thereof, especially in the appended claims, unless otherwise expressly stated, should be construed as open ended as opposed to limiting. As examples of the foregoing, the term 'including' should be read to mean 'including, without limitation,' 'including but not limited to,' or the like; the term 'comprising' as used herein is synonymous with 'including,' 'containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide exemplary instances of the item in discussion, not an exhaustive or limiting list thereof; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular embodiment. In addition, the term "comprising" is to be interpreted synonymously with the phrases "having at least" or "including at least". When used in the context of a process, the term "comprising" means that the process includes at least the recited steps, but may include additional steps. When used in the context of a compound, composition or device, the term "comprising" means that the compound, composition or device includes at least the recited features or components, but may also include additional features or components. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

It is understood that, in any compound described herein having one or more chiral centers, if an absolute stereochemistry is not expressly indicated, then each center may independently be of R-configuration or S-configuration or a mixture thereof. Thus, the compounds provided herein may be enantiomerically pure, enantiomerically enriched, racemic mixture, diastereomerically pure, diastereomerically enriched, or a stereoisomeric mixture. In addition it is understood that, in any compound described herein having one or more double bond(s) generating geometrical isomers that can be defined as E or Z, each double bond may independently be E or Z a mixture thereof.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present embodiments. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope of the embodiments are approximations, the numerical values set forth in the specific examples are reported as precisely as possible. Any numerical value, however, inherently contains certain errors necessarily resulting from the standard deviation found in their respective testing measurements.

### CSA Compound Synthesis:

The methods disclosed herein may be as described below, or by modification of these methods. Ways of modifying the methodology include, among others, temperature, solvent, reagents etc., known to those skilled in the art. In general, during any of the processes for preparation disclosed herein, it may be necessary and/or desirable to protect sensitive or reactive groups on any of the molecules concerned. This may be achieved by means of conventional protecting groups, such as those described *in* Protective Groups in Organic Chemistry (ed. J.F.W. McOmie, Plenum Press, 1973); and P.G.M. Green, T.W. Wutts, Protecting Groups in Organic Synthesis (3rd ed.) Wiley, New York (1999). The protecting groups may be removed at a convenient subsequent stage using methods known from the art. Synthetic chemistry transformations useful in synthesizing applicable compounds are known in the art and include e.g. those described in R. Larock, Comprehensive Organic Transformations, VCH Publishers, 1989, or L. Paquette, ed., Encyclopedia of Reagents for Organic Synthesis, John Wiley and Sons, 1995, which are both hereby incorporated herein by reference in their entirety. The routes shown and described herein are illustrative only and are not intended, nor are they to be construed, to limit the scope of the claims in any manner whatsoever. Those skilled in the art will be able to recognize modifications of the disclosed syntheses and to devise alternate routes based on the disclosures herein; all such modifications and alternate routes are within the scope of the claims.

An exemplary but non-limiting general synthetic scheme for preparing a compound of Formula (I) is shown in Scheme A-1, below. Unless otherwise indicated, the variable definitions are as above for Formula (I). This process starts with a cholic acid (1). Treatment of (1) with secondary amine R₁R₂NH under amide bond forming conditions affords intermediate (2). Amide bond forming reagents include, but are not limited to, EDAC [N-(3-Dimethylaminopropyl)-N'-ethylcarbodiimide hydrochloride] in the presence of HOBT (1-hydroxybenzotriazole), or HATU [N,N,N',N'-Tetramethyl-O-(7-azabenzotriazol-1-yl)uronium hexafluorophosphate) in the presence of diisopropylethylamine, and the like.

In some embodiments, R₁ and R₂ are independently selected from the group consisting of hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, and a suitable amine protecting group, provided that at least one of R₁ or R₂ is not a hydrogen.

Compound (2) is treated with an alkoxyacroylonitrile reagent in the presence of acid and a phase transfer catalyst to afford compounds of Formula (3). In some embodiments, the acid is an organic acid. In some embodiments, the acid is an inorganic acid. In some embodiments, the acid is used in catalytic amounts. In some embodiments, the acid is used in stoichiometric amounts. In some embodiments, the acid is used in greater than stoichiometric amounts. In some embodiments, the phase transfer catalyst is tetrabutylammonium iodide. In some embodiments, the phase transfer catalyst is tetrabutylammonium bromide.

Compound (3) is then subjected to reducing conditions to afford compounds of Formula (I). Suitable reducing conditions include, but are not limited to RedAl, lithium aluminum hydride, lithium borohydride, or treatment with hydrogen in the presence of a suitable metal catalyst or treatment with silyl hydrides in the presence of a suitable metal catalyst. Suitable metal catalysts are known in the art.

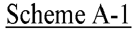

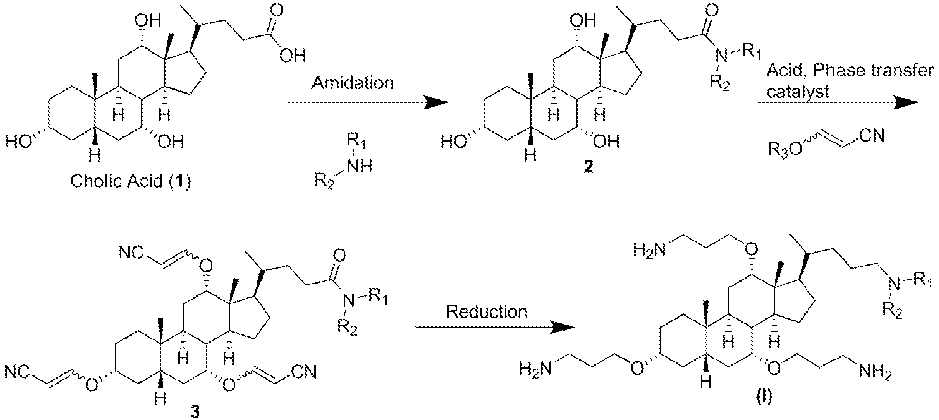

An exemplary but non-limiting general synthetic scheme for preparing a compound of Formula (II) is shown in Scheme A-2, below. Unless otherwise indicated, the variable definitions are as above. This process starts with a cholic acid (1). Treatment of (1) with secondary amine R₁R₂NH under amide bond forming conditions affords intermediate (2). Amide bond forming reagents include, but are not limited to, EDAC [N-(3-Dimethylaminopropyl)-N'-ethylcarbodiimide hydrochloride] in the presence of HOBT (1-hydroxybenzotriazole), or HATU [N,N,N',N'-Tetramethyl-O-(7-azabenzotriazol-1-yl)uronium hexafluorophosphate) in the presence of diisopropylethylamine, and the like.

In some embodiments, R₁ and R₂ are independently selected from the group consisting of hydrogen, optionally substituted C₁-C₁₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, and a suitable amine protecting group, provided that at least one of R₁ or R₂ is not a hydrogen.

Compound (2) is treated with an alkoxyacroylonitrile reagent in the presence of acid and a phase transfer catalyst to afford compounds of Formula (3). In some embodiments, the acid is an organic acid. In some embodiments, the acid is an inorganic acid. In some embodiments, the acid is used in catalytic amounts. In some embodiments, the acid is used in stoichiometric amounts. In some embodiments, the acid is used in greater than stoichiometric amounts. In some embodiments, the phase transfer catalyst is tetrabutylammonium iodide. In some embodiments, the phase transfer catalyst is tetrabutylammonium bromide.

Compound (3) is then subjected to reducing conditions to afford compounds of Formula (II). Suitable reducing conditions include, but are not limited to RedAl, lithium aluminum hydride, lithium borohydride, or treatment with hydrogen in the presence of a suitable metal catalyst or treatment with silyl hydrides in the presence of a suitable metal catalyst. Suitable metal catalysts are known in the art. The reduction is carried out such that the C-amido group of compound (3) is preferentially not reduced whereas the cyano groups of compound (3) are preferentially reduced.

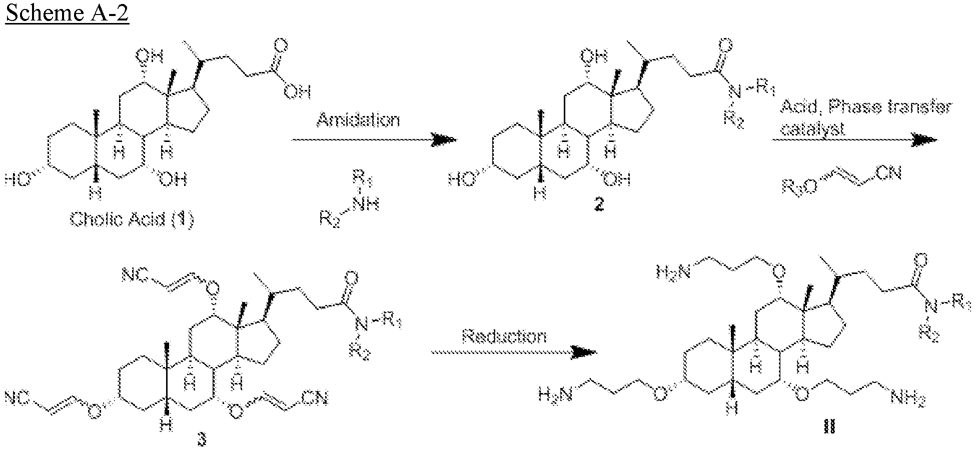

## Claims

1. A method of making a compound of Formula (I) or (II), comprising the steps of:
(a) reacting a compound of Formula (1) and R₁R₂-NH 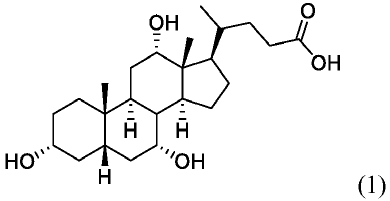 to form a compound of Formula (2): 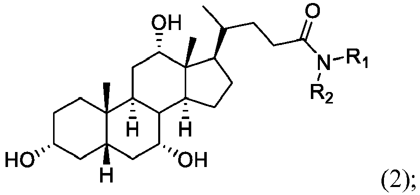
(b) reacting a compound of Formula (2) with an compound of Formula (A), 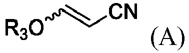 in the presence of acid and a phase transfer catalyst, to form a compound of Formula (3): 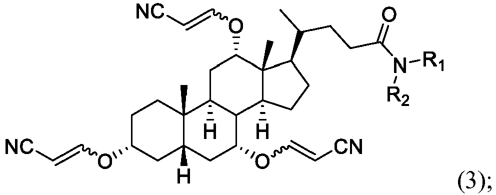 and
(c) subjecting a compound of Formula (3) to reducing conditions to form a compound of Formula (I): 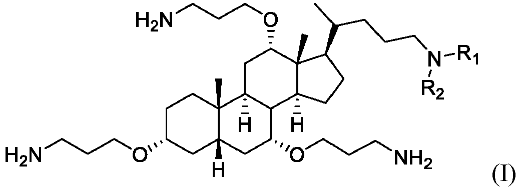 or a compound of Formula (II): 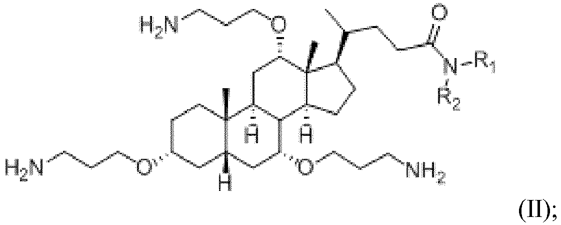 wherein:
R₁ and R₂ are independently selected from the group consisting of hydrogen, optionally substituted C₁-C₂₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, optionally substituted amido, and a suitable amine protecting group; and
R₃ is selected from the group consisting of optionally substituted C₁-C₂₄ alkyl, optionally substituted C₂-C₂₄ alkenyl, optionally substituted C₂-C₂₄ alkynyl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, and optionally substituted (5 to 10 membered heterocyclyl)-C₁-C₆ alkyl,
the method is carried out at a temperature of at least at least 0°C, at least 10°C, at least 20°C, at least 30°C, at least 40°C, at least 50°C, at least 60°C, at least 70°C, at least 80°C, at least 90°C, at least 100°C, at least 110°C, at least 120°C, at least 130°C, at least 140°C, at least 150°C, at least 160°C, at least 170°C, or at least 180°C.

2. A method of making a compound of Formula (I), comprising the steps of:
(a) reacting a compound of Formula (1) and R₁R₂-NH 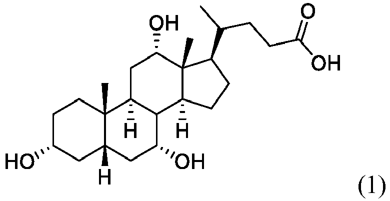 to form a compound of Formula (2): 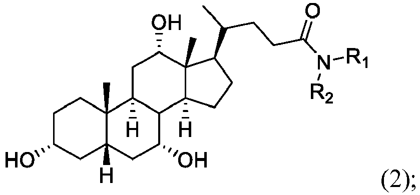
(b) reacting a compound of Formula (2) with an compound of Formula (A), 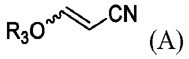 in the presence of acid and a phase transfer catalyst, to form a compound of Formula (3): 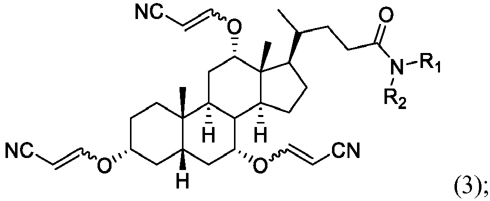 and
(c) subjecting a compound of Formula (3) to reducing conditions to form a compound of Formula (I): 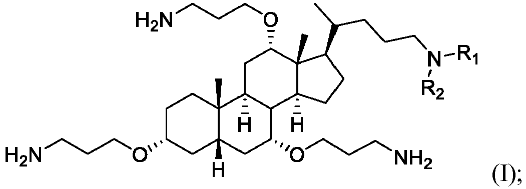 wherein:
R₁ and R₂ are independently selected from the group consisting of hydrogen, optionally substituted C₁-C₂₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, optionally substituted amido, and a suitable amine protecting group; and
R₃ is selected from the group consisting of optionally substituted C₁-C₂₄ alkyl, optionally substituted C₂-C₂₄ alkenyl, optionally substituted C₂-C₂₄ alkynyl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, and optionally substituted (5 to 10 membered heterocyclyl)-C₁-C₆ alkyl.

3. The method of claim 1 or 2, wherein R₁ is hydrogen and R₂ is selected from the group consisting of hydrogen, optionally substituted C₁-C₂₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, optionally substituted (5 to 10 membered heterocyclyl)-C₁-C₆ alkyl, and a suitable amine protecting group.

4. The method of any one of claims 1-3, wherein R₁ is hydrogen and R₂ is selected from the group consisting of optionally substituted C₁-C₂₄ alkyl, optionally substituted C₂-C₂₄ alkenyl, optionally substituted C₂-C₂₄ alkynyl, optionally substituted C₆ or C₁₀ aryl, optionally substituted 5 to 10 membered heteroaryl, optionally substituted 5 to 10 membered heterocyclyl, optionally substituted C₇₋₁₃ aralkyl, optionally substituted (5 to 10 membered heteroaryl)-C₁-C₆ alkyl, optionally substituted C₃₋₁₀ carbocyclyl, optionally substituted C₄₋₁₀ (carbocyclyl)alkyl, and optionally substituted (5 to 10 membered heterocyclyl)-C₁-C₆ alkyl.

5. The method of any one of claims 1-4, wherein R₁ is hydrogen and R₂ is optionally substituted C₁-C₂₄ alkyl.

6. The method of any one of claims 1-5, wherein the compound of Formula (I) is selected from the group consisting of:

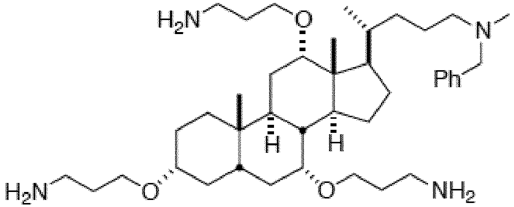

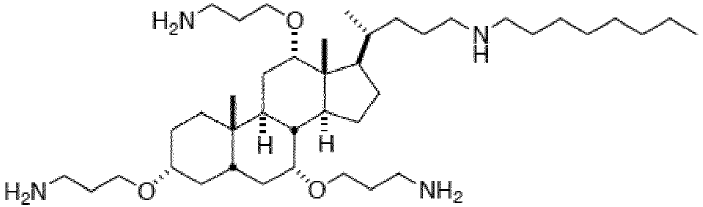

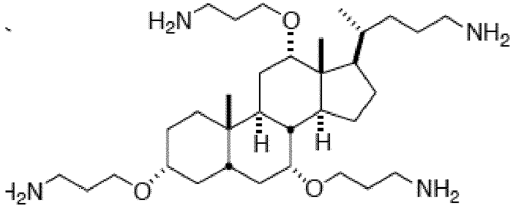

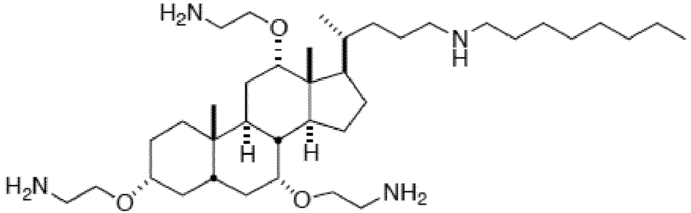

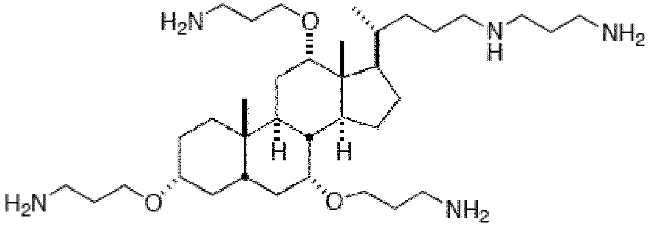

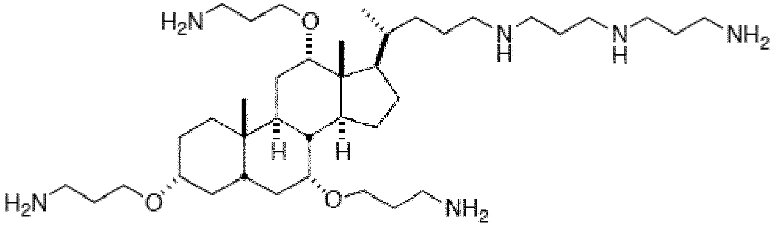

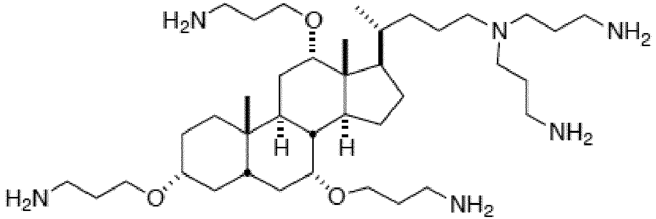

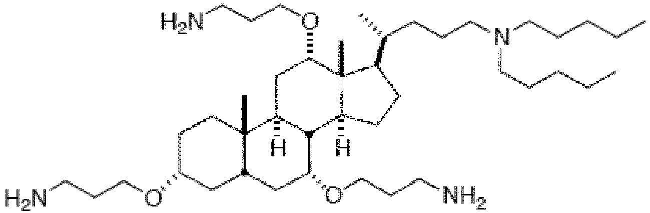

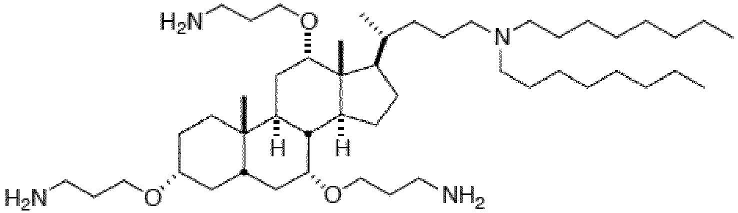

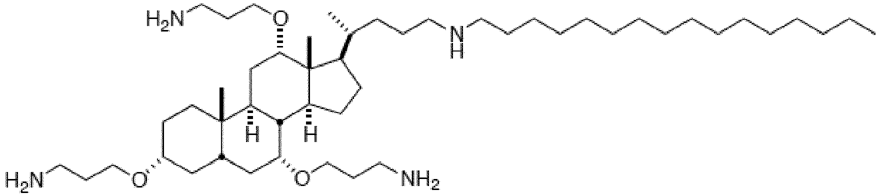

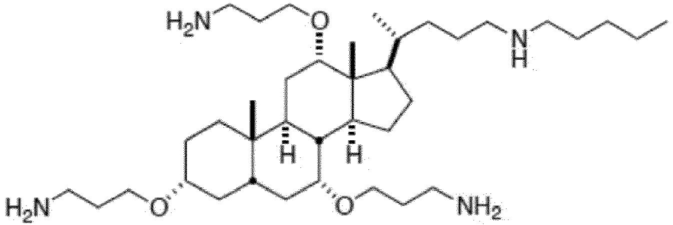

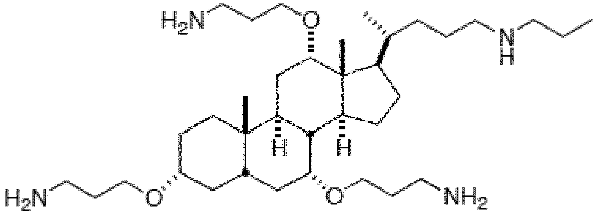

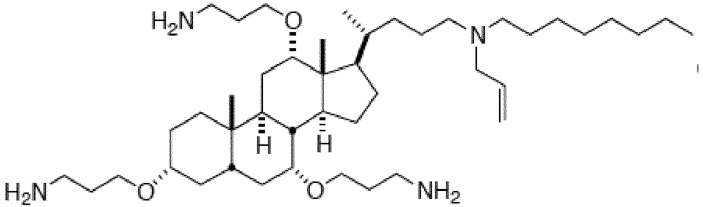

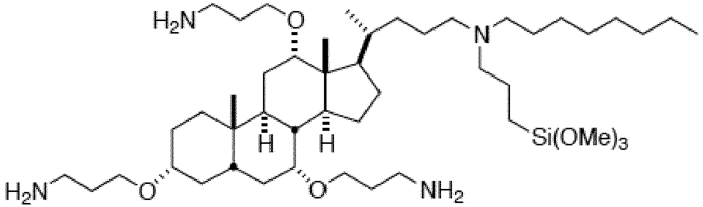

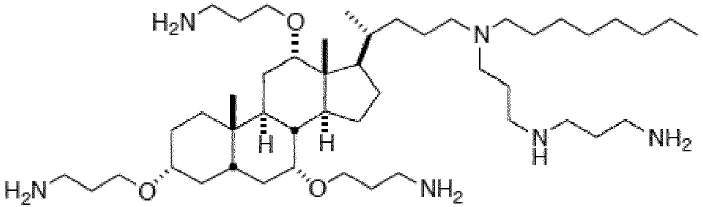

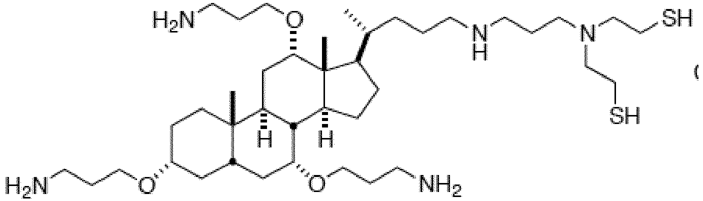

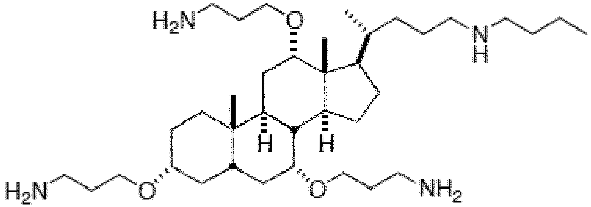

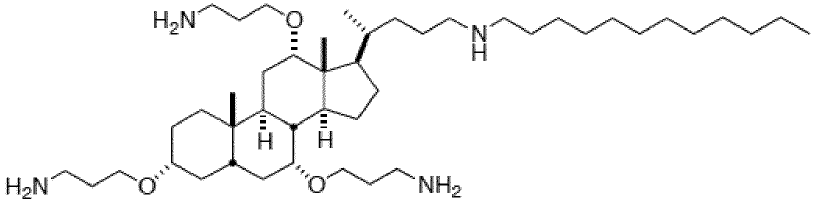

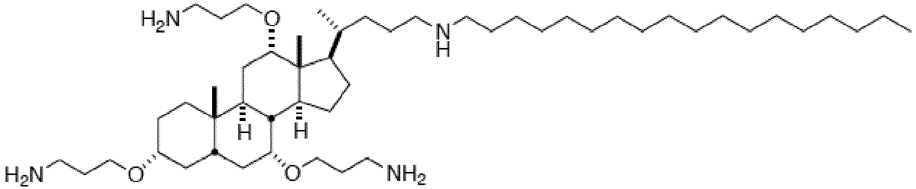

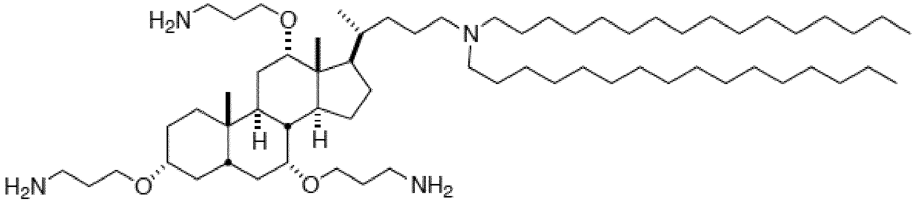

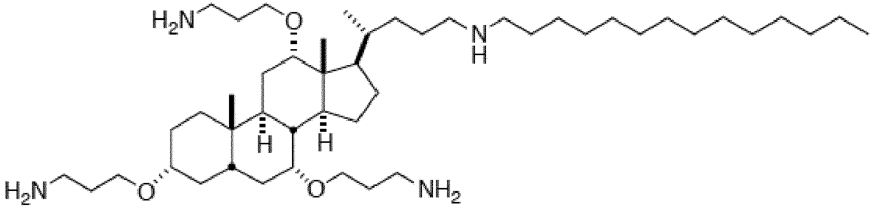

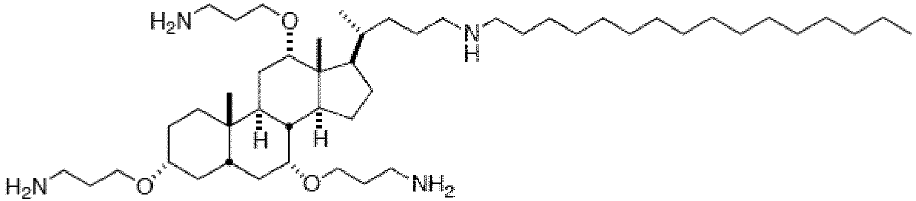

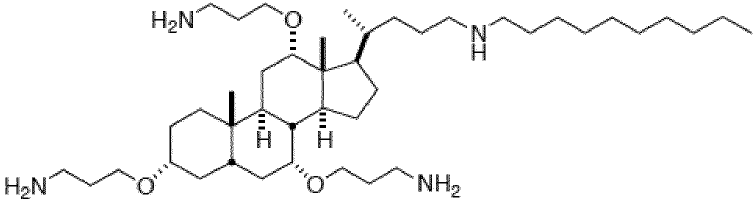

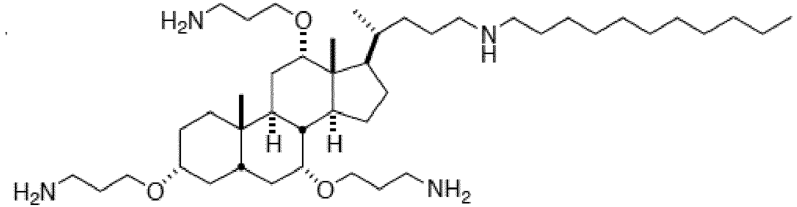

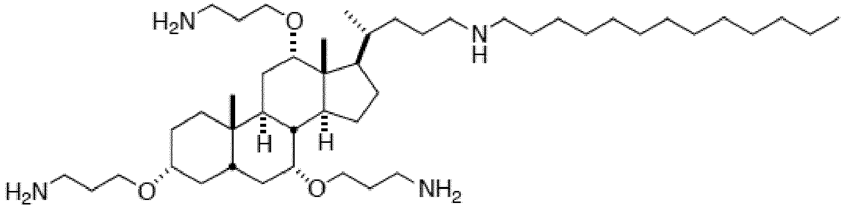

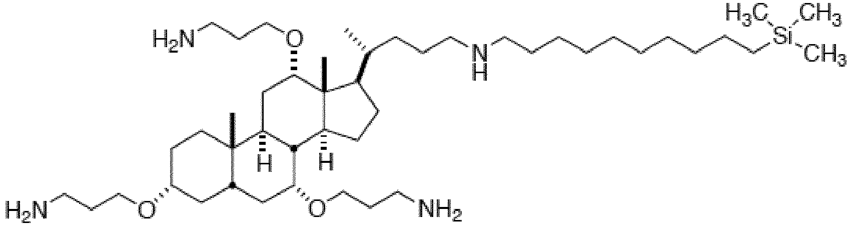

7. The method of Claim 1, wherein the compound of Formula (II) is selected from the group consisting of: 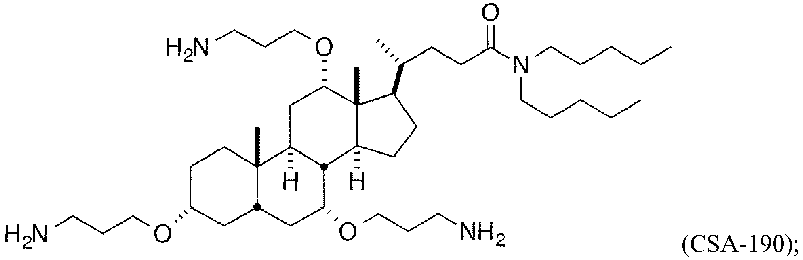 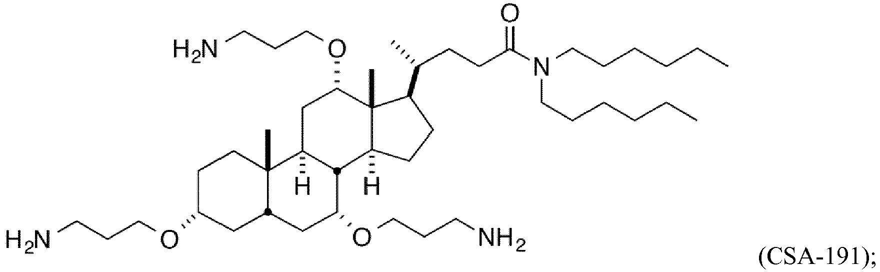 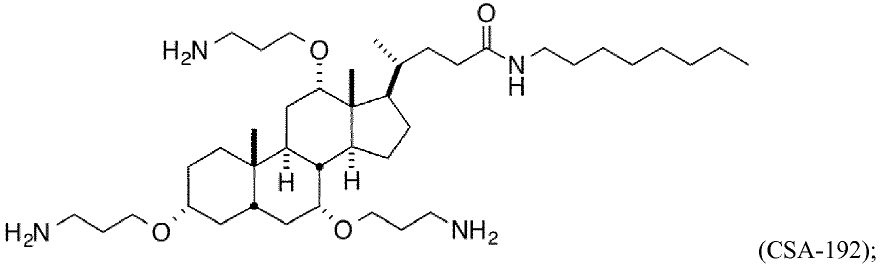 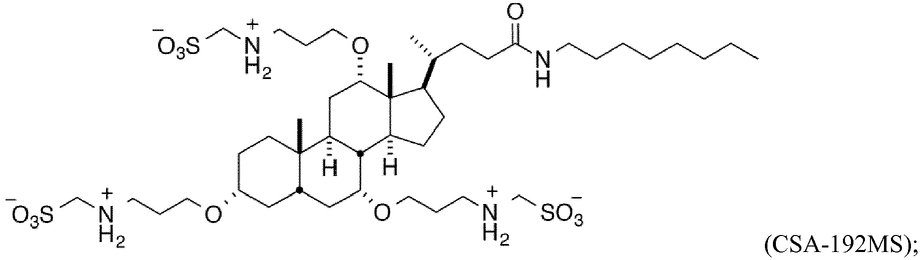 and salts thereof.

8. The method of any one of claims 1-7, wherein the method is carried out in one or more solvents selected from the group consisting of water, acetic acid, formic acid, hydrochloric acid, hydrobromic acid, DMF, DMSO, DCM, NMP, chloroform, THF, 2-methyl-THF, benzene, toluene, trifluorotoluene, dioxane, methanol, ethanol, ethyl acetate, acetone, or any combination thereof.

9. The method of any one of claims 1-8, wherein the method is accomplished in less than 36 hours, less than 35 hours, less than 34 hours, less than 33 hours, less than 32 hours, less than 31 hours, less than 30 hours, less than 29 hours, less than 28 hours, less than 27 hours, less than 26 hours, less than 25 hours, less than 24 hours, less than 23 hours, less than 22 hours, less than 21 hours, less than 20 hours, less than 19 hours, less than 18 hours, less than 17 hours, less than 16 hours, less than 15 hours, less than 14 hours, less than 13 hours, less than 12 hours, less than 11 hours, less than 10 hours, less than 9 hours, less than 8 hours, less than 7 hours, less than 6 hours, less than 5 hours, less than 4 hours, less than 3 hours, less than 2 hours, or less than 1 hour.

10. The method of any one of claims 1-9, wherein the method provides at least one gram, at least 10 grams, and least 100 grams, at least 500 grams, at least one kilogram, at least 10 kilograms, at least 50 kilograms, at least 100 kilogram, or at least 500 kilograms, of a compound of Formula (I) or a compound of Formula (II).

11. The method of any one of claims 1-10, wherein the method is substantially performed at ambient pressure.

12. The method of any one of claims 1-11, wherein the method does not deliberately exclude the presence of oxygen or is substantially performed in a non-oxygen-containing atmosphere.

13. The method of any one of claims 1-12, wherein the method is performed with a mixing rate of at least 1 rpm, at least 10 rpms, at least 20 rpms, at least 30 rpms, at least 40 rpms, at least 50 rpms, at least 60 rpms, at least 70 rpms, at least 80 rpms, at least 90 rpms, at least 100 rpms, at least 110 rpms, at least 120 rpms, at least 200 rpms, or at least 500 rpms.

## Patentansprüche

1. Verfahren zur Herstellung einer Verbindung der Formel (I) oder (II), umfassend die folgenden Schritte:
(a) Umsetzen einer Verbindung der Formel (1) und von R₁R₂-NH 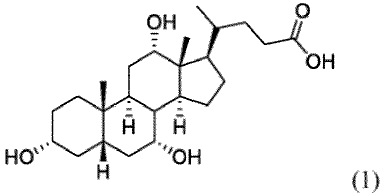 zur Bildung einer Verbindung der Formel (2): 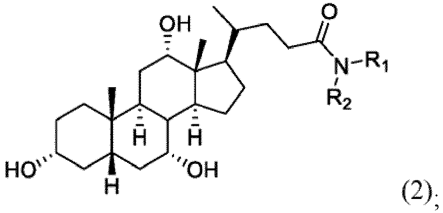
(b) Umsetzen einer Verbindung der Formel (2) mit einer Verbindung der Formel (A) 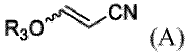 in Gegenwart einer Säure und eines Phasentransferkatalysators zur Bildung einer Verbindung der Formel (3): 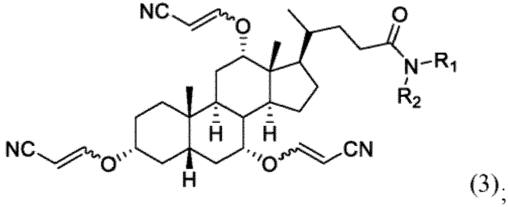 und
(c) Aussetzen einer Verbindung der Formel (3) an reduzierende Bedingungen zur Bildung einer Verbindung der Formel (I): 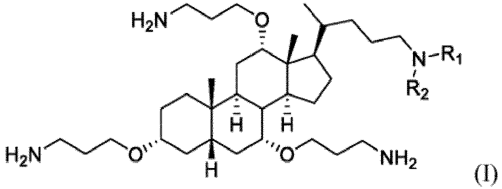 oder einer Verbindung der Formel (II): 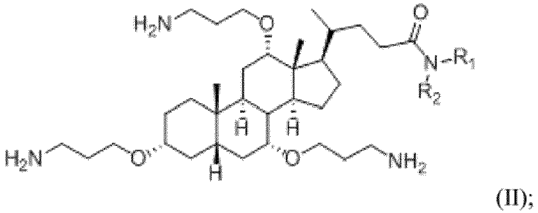 wobei:
R₁ und R₂ unabhängig voneinander ausgewählt sind aus der Gruppe, bestehend aus Wasserstoff, gegebenenfalls substituiertem C₁-C₂₄-Alkyl, gegebenenfalls substituiertem C₂-C₂₄-Alkenyl, gegebenenfalls substituiertem C₂-C₂₄-Alkinyl, gegebenenfalls substituiertem C₆- oder C₁₀-Aryl, gegebenenfalls substituiertem 5- bis 10-gliedrigem Heteroaryl, gegebenenfalls substituiertem 5- bis 10-gliedrigem Heterocyclyl, gegebenenfalls substituiertem C₇₋₁₃-Aralkyl, gegebenenfalls substituiertem (5-bis 10-gliedrigem Heteroaryl)-C₁-C₆-alkyl, gegebenenfalls substituiertem C₃₋₁₀-Carbocyclyl, gegebenenfalls substituiertem C₄₋₁₀-(Carbocyclyl)alkyl, gegebenenfalls substituiertem (5-bis 10-gliedrigem Heterocyclyl)-C₁-C₆-alkyl, gegebenenfalls substituiertem Amido und einer geeigneten Aminschutzgruppe; und
R₃ ausgewählt ist aus der Gruppe, bestehend aus gegebenenfalls substituiertem C₁-C₂₄-Alkyl, gegebenenfalls substituiertem C₂-C₂₄-Alkenyl, gegebenenfalls substituiertem C₂-C₂₄-Alkinyl, gegebenenfalls substituiertem 5- bis 10-gliedrigem Heterocyclyl, gegebenenfalls substituiertem C₇₋₁₃-Aralkyl, gegebenenfalls substituiertem (5- bis 10-gliedrigem Heteroaryl)-C₁-C₆-alkyl, gegebenenfalls substituiertem C₃₋₁₀-Carbocyclyl, gegebenenfalls substituiertem C₄₋₁₀-(Carbocyclyl)alkyl und gegebenenfalls substituiertem (5-bis 10-gliedrigem Heterocyclyl)-C₁-C₆-alkyl,
wobei das Verfahren bei einer Temperatur von mindestens 0°C, mindestens 10°C, mindestens 20°C, mindestens 30°C, mindestens 40°C, mindestens 50°C, mindestens 60°C, mindestens 70°C, mindestens 80°C, mindestens 90°C, mindestens 100°C, mindestens 110°C, mindestens 120°C, mindestens 130°C, mindestens 140°C, mindestens 150°C, mindestens 160°C, mindestens 170°C oder mindestens 180°C durchgeführt wird.

2. Verfahren zur Herstellung einer Verbindung der Formel (I), umfassend die folgenden Schritte:
(a) Umsetzen einer Verbindung der Formel (1) und von R₁R₂-NH 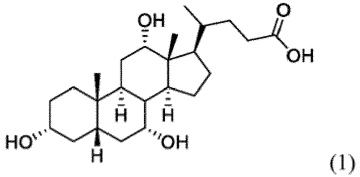 zur Bildung einer Verbindung der Formel (2): 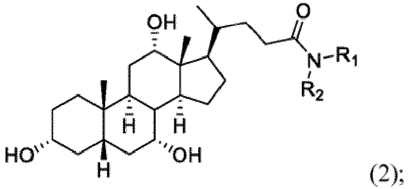
(b) Umsetzen einer Verbindung der Formel (2) mit einer Verbindung der Formel (A) 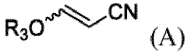 in Gegenwart einer Säure und eines Phasentransferkatalysators zur Bildung einer Verbindung der Formel (3): 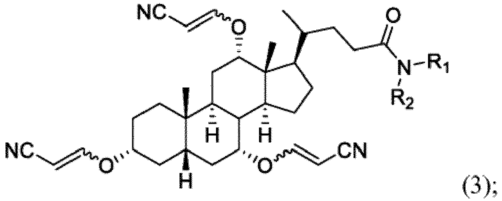 und
(c) Aussetzen einer Verbindung der Formel (3) an reduzierende Bedingungen zur Bildung einer Verbindung der Formel (I): 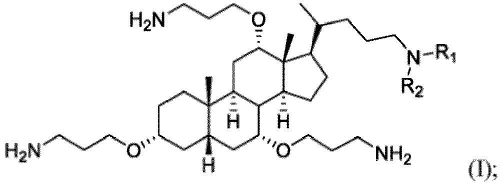 wobei:
R₁ und R₂ unabhängig voneinander ausgewählt sind aus der Gruppe, bestehend aus Wasserstoff, gegebenenfalls substituiertem C₁-C₂₄-Alkyl, gegebenenfalls substituiertem C₂-C₂₄-Alkenyl, gegebenenfalls substituiertem C₂-C₂₄-Alkinyl, gegebenenfalls substituiertem C₆- oder C₁₀-Aryl, gegebenenfalls substituiertem 5- bis 10-gliedrigem Heteroaryl, gegebenenfalls substituiertem 5- bis 10-gliedrigem Heterocyclyl, gegebenenfalls substituiertem C₇₋₁₃-Aralkyl, gegebenenfalls substituiertem (5-bis 10-gliedrigem Heteroaryl)-C₁-C₆-alkyl, gegebenenfalls substituiertem C₃₋₁₀-Carbocyclyl, gegebenenfalls substituiertem C₄₋₁₀-(Carbocyclyl)alkyl, gegebenenfalls substituiertem (5-bis 10-gliedrigem Heterocyclyl)-C₁-C₆-alkyl, gegebenenfalls substituiertem Amido und einer geeigneten Aminschutzgruppe; und
R₃ ausgewählt ist aus der Gruppe, bestehend aus gegebenenfalls substituiertem C₁-C₂₄-Alkyl, gegebenenfalls substituiertem C₂-C₂₄-Alkenyl, gegebenenfalls substituiertem C₂-C₂₄-Alkinyl, gegebenenfalls substituiertem 5- bis 10-gliedrigem Heterocyclyl, gegebenenfalls substituiertem C₇₋₁₃-Aralkyl, gegebenenfalls substituiertem (5- bis 10-gliedrigem Heteroaryl)-C₁-C₆-alkyl, gegebenenfalls substituiertem C₃₋₁₀-Carbocyclyl, gegebenenfalls substituiertem C₄₋₁₀-(Carbocyclyl)alkyl und gegebenenfalls substituiertem (5-bis 10-gliedrigem Heterocyclyl)-C₁-C₆-alkyl.

3. Verfahren nach Anspruch 1 oder 2, wobei R₁ Wasserstoff ist und R₂ ausgewählt ist aus der Gruppe, bestehend aus Wasserstoff, gegebenenfalls substituiertem C₁-C₂₄-Alkyl, gegebenenfalls substituiertem C₂-C₂₄-Alkenyl, gegebenenfalls substituiertem C₂-C₂₄-Alkinyl, gegebenenfalls substituiertem C₆- oder C₁₀-Aryl, gegebenenfalls substituiertem 5-bis 10-gliedrigem Heteroaryl, gegebenenfalls substituiertem 5- bis 10-gliedrigem Heterocyclyl, gegebenenfalls substituiertem C₇₋₁₃-Aralkyl, gegebenenfalls substituiertem (5-bis 10-gliedrigem Heteroaryl)-C₁-C₆-alkyl, gegebenenfalls substituiertem C₃₋₁₀-Carbocyclyl, gegebenenfalls substituiertem C₄₋₁₀-(Carbocyclyl)alkyl, gegebenenfalls substituiertem (5- bis 10-gliedrigem Heterocyclyl)-C₁-C₆-alkyl und einer geeigneten Aminschutzgruppe.

4. Verfahren nach einem der Ansprüche 1-3, wobei R₁ Wasserstoff ist und R₂ ausgewählt ist aus der Gruppe, bestehend aus gegebenenfalls substituiertem C₁-C₂₄-Alkyl, gegebenenfalls substituiertem C₂-C₂₄-Alkenyl, gegebenenfalls substituiertem C₂-C₂₄-Alkinyl, gegebenenfalls substituiertem C₆- oder C₁₀-Aryl, gegebenenfalls substituiertem 5-bis 10-gliedrigem Heteroaryl, gegebenenfalls substituiertem 5- bis 10-gliedrigem Heterocyclyl, gegebenenfalls substituiertem C₇₋₁₃-Aralkyl, gegebenenfalls substituiertem (5-bis 10-gliedrigem Heteroaryl)-C₁-C₆-alkyl, gegebenenfalls substituiertem C₃₋₁₀-Carbocyclyl, gegebenenfalls substituiertem C₄₋₁₀-(Carbocyclyl)alkyl und gegebenenfalls substituiertem (5-bis 10-gliedrigem Heterocyclyl)-C₁-C₆-alkyl.

5. Verfahren nach einem der Ansprüche 1-4, wobei R₁ Wasserstoff ist und R₂ gegebenenfalls substituiertes C₁-C₂₄-Alkyl ist.

6. Verfahren nach einem der Ansprüche 1-5, wobei die Verbindung der Formel (I) ausgewählt ist aus der Gruppe, bestehend aus:

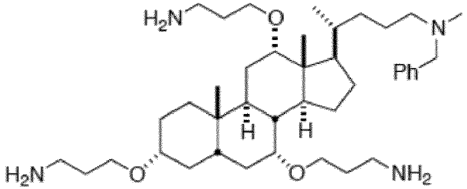

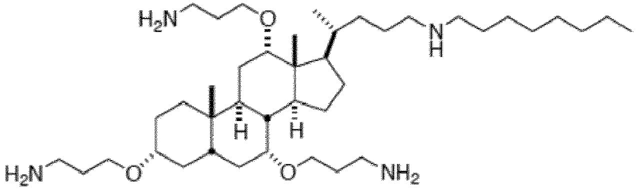

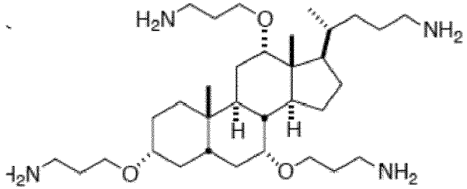

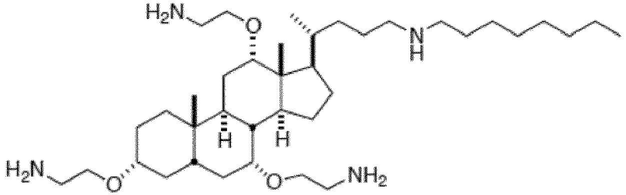

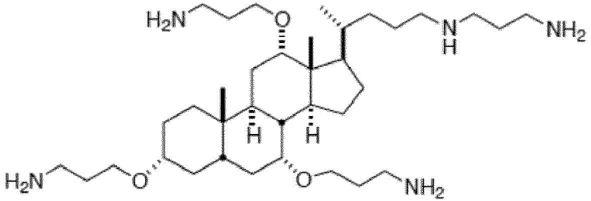

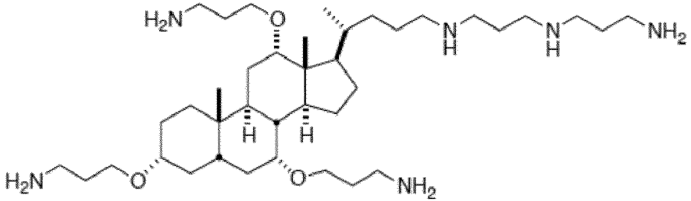

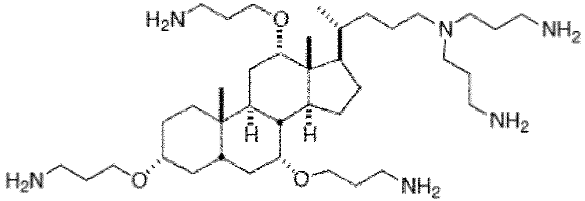

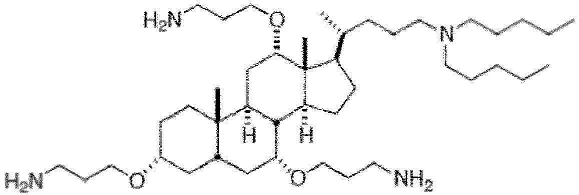

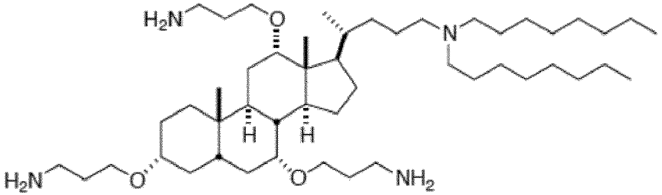

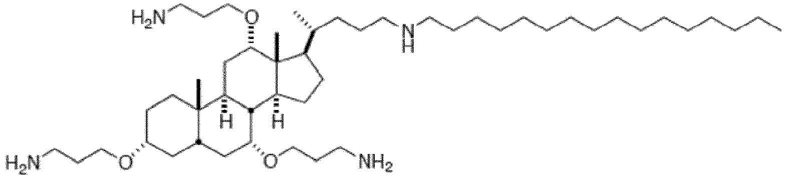

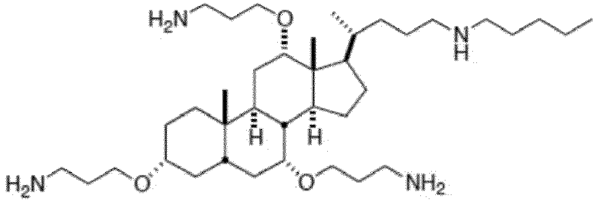

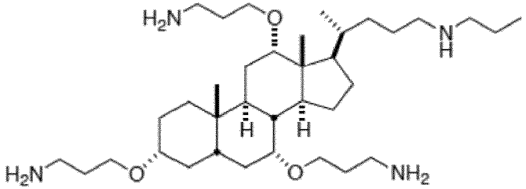

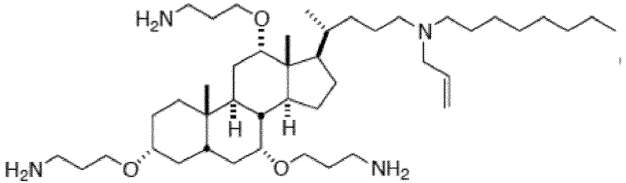

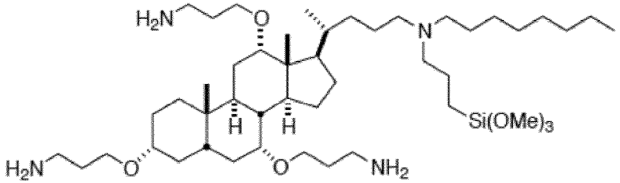

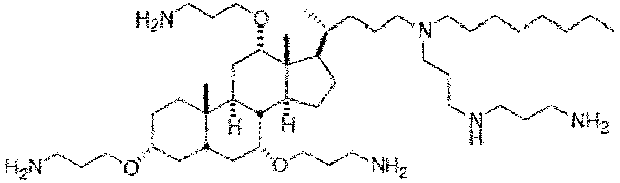

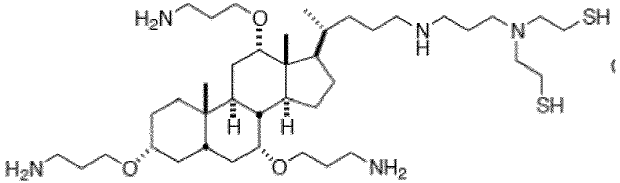

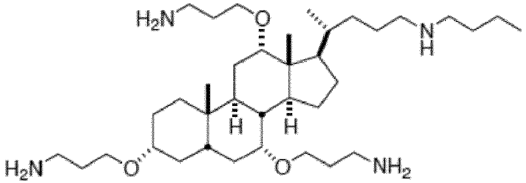

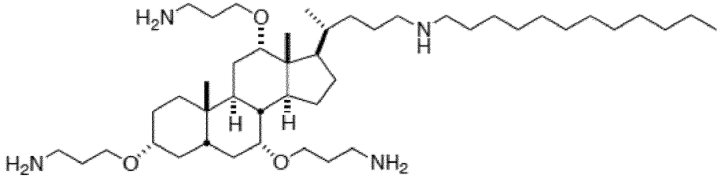

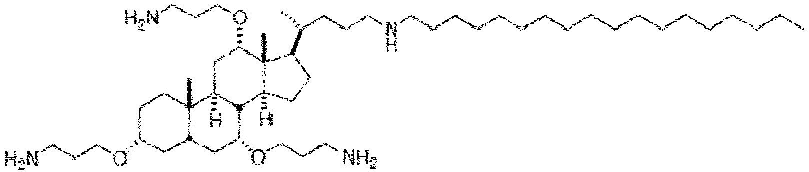

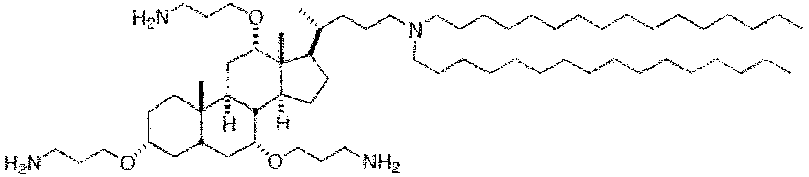

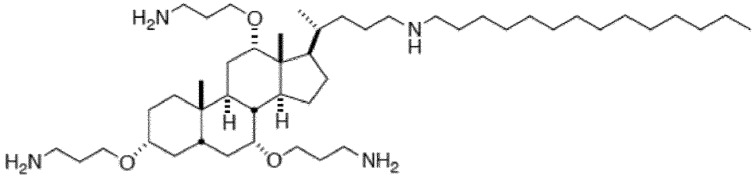

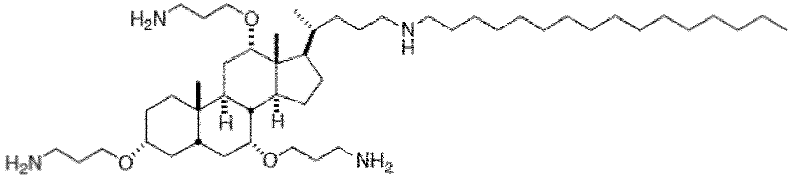

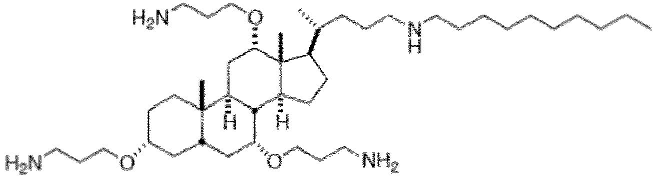

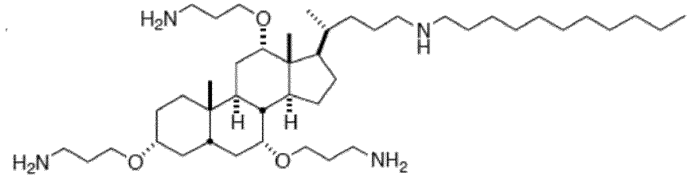

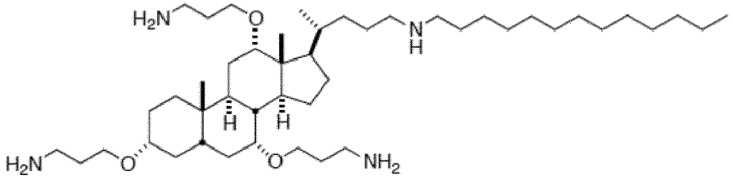

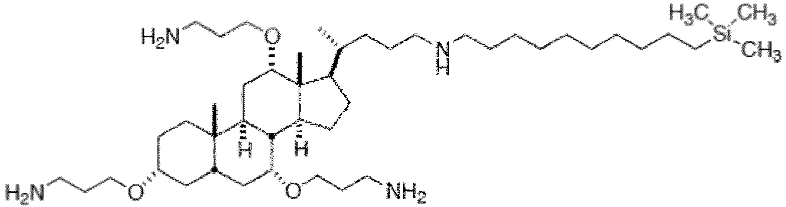

7. Verfahren nach Anspruch 1, wobei die Verbindung der Formel (II) ausgewählt ist aus der Gruppe, bestehend aus: 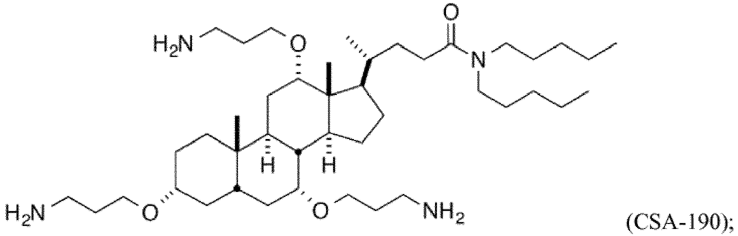 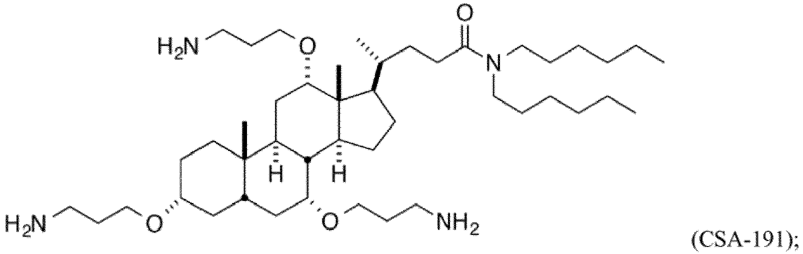 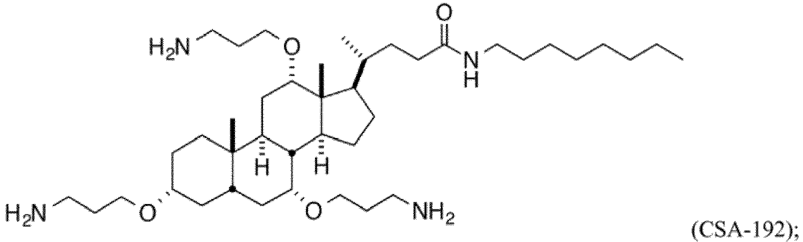 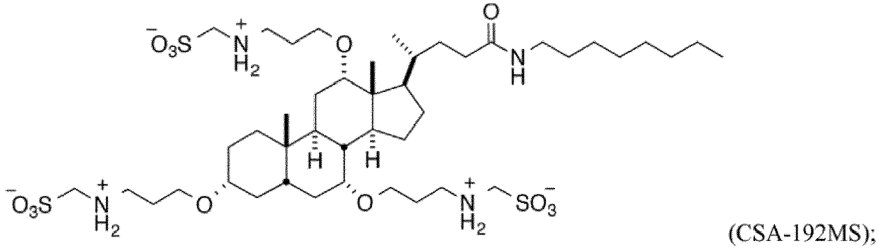 und Salzen davon.

8. Verfahren nach einem der Ansprüche 1-7, wobei das Verfahren in einem oder mehreren Lösungsmitteln, ausgewählt aus der Gruppe, bestehend aus Wasser, Essigsäure, Ameisensäure, Salzsäure, Bromwasserstoffsäure, DMF, DMSO, DCM, NMP, Chloroform, THF, 2-Methyl-THF, Benzol, Toluol, Trifluortoluol, Dioxan, Methanol, Ethanol, Ethylacetat, Aceton oder einer beliebige Kombination davon, durchgeführt wird.

9. Verfahren nach einem der Ansprüche 1-8, wobei das Verfahren in weniger als 36 Stunden, weniger als 35 Stunden, weniger als 34 Stunden, weniger als 33 Stunden, weniger als 32 Stunden, weniger als 31 Stunden, weniger als 30 Stunden, weniger als 29 Stunden, weniger als 28 Stunden, weniger als 27 Stunden, weniger als 26 Stunden, weniger als 25 Stunden, weniger als 24 Stunden, weniger als 23 Stunden, weniger als 22 Stunden, weniger als 21 Stunden, weniger als 20 Stunden, weniger als 19 Stunden, weniger als 18 Stunden, weniger als 17 Stunden, weniger als 16 Stunden, weniger als 15 Stunden, weniger als 14 Stunden, weniger als 13 Stunden, weniger als 12 Stunden, weniger als 11 Stunden, weniger als 10 Stunden, weniger als 9 Stunden, weniger als 8 Stunden, weniger als 7 Stunden, weniger als 6 Stunden, weniger als 5 Stunden, weniger als 4 Stunden, weniger als 3 Stunden, weniger als 2 Stunden oder weniger als 1 Stunde durchgeführt wird.

10. Verfahren nach einem der Ansprüche 1-9, wobei das Verfahren mindestens ein Gramm, mindestens 10 Gramm, mindestens 100 Gramm, mindestens 500 Gramm, mindestens ein Kilogramm, mindestens 10 Kilogramm, mindestens 50 Kilogramm, mindestens 100 Kilogramm oder mindestens 500 Kilogramm einer Verbindung der Formel (I) oder einer Verbindung der Formel (II) bereitstellt.

11. Verfahren nach einem der Ansprüche 1-10, wobei das Verfahren im Wesentlichen bei Umgebungsdruck durchgeführt wird.

12. Verfahren nach einem der Ansprüche 1-11, wobei das Verfahren das Vorhandensein von Sauerstoff nicht vorsätzlich ausschließt oder im Wesentlichen in einer nicht sauerstoffhaltigen Atmosphäre durchgeführt wird.

13. Verfahren nach einem der Ansprüche 1-12, wobei das Verfahren mit einer Mischgeschwindigkeit von mindestens 1 U/min, mindestens 10 U/min, mindestens 20 U/min, mindestens 30 U/min, mindestens 40 U/min, mindestens 50 U/min, mindestens 60 U/min, mindestens 70 U/min, mindestens 80 U/min, mindestens 90 U/min, mindestens 100 U/min, mindestens 110 U/min, mindestens 120 U/min, mindestens 200 U/min oder mindestens 500 U/min durchgeführt wird.

## Revendications

1. Méthode de fabrication d'un composé de Formule (I) ou (II), comprenant les étapes consistant à :
(a) faire réagir un composé de Formule (1) et R₁R₂-NH 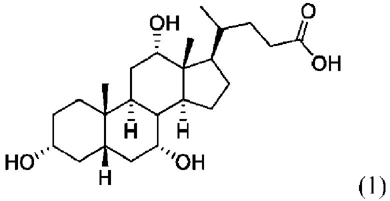 pour former un composé de Formule (2) : 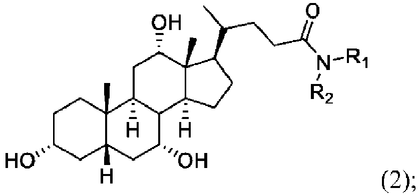
(b) faire réagir un composé de Formule (2) avec un composé de Formule (A), 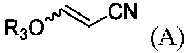 en présence d'acide et d'un catalyseur de transfert de phase, pour former un composé de Formule (3) : 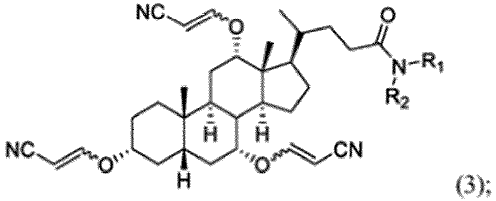 et
(c) soumettre un composé de Formule (3) à des conditions réductrices pour former un composé de Formule (I) : 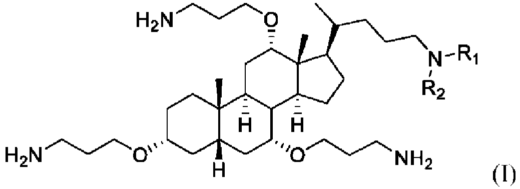 ou un composé de Formule (II) : 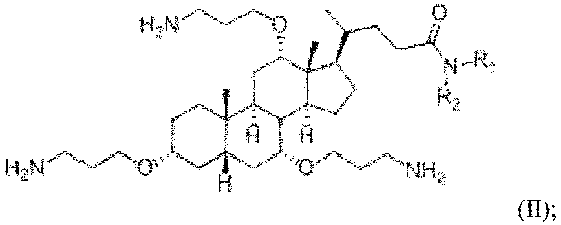 où :
R₁ et R₂ sont indépendamment choisis parmi le groupe constitué d'hydrogène, alkyle en C₁-C₁₄ facultativement substitué, alcényle en C₂-C₂₄ facultativement substitué, alcynyle en C₂-C₂₄ facultativement substitué, aryle en C₆ ou C₁₀ facultativement substitué, hétéroaryle de 5 à 10 éléments facultativement substitué, hétérocyclyle de 5 à 10 éléments facultativement substitué, aralkyle en C₇₋₁₃ facultativement substitué, (hétéroaryle de 5 à 10 éléments)-alkyle en C₁-C₆ facultativement substitué, carbocyclyle en C₃₋₁₀ facultativement substitué, (carbocyclyl)alkyle en C₄₋₁₀ facultativement substitué, (hétérocyclyle de 5 à 10 éléments)-alkyle en C₁-C₆ facultativement substitué, amido facultativement substitué, et un groupe protecteur d'amine approprié ; et
R₃ est choisi parmi le groupe constitué d'alkyle en C₁-C₂₄ facultativement substitué, alcényle en C₂-C₂₄ facultativement substitué, alcynyle en C₂-C₂₄ facultativement substitué, hétérocyclyle de 5 à 10 éléments facultativement substitué, aralkyle en C₇₋₁₃ facultativement substitué, (hétéroaryle de 5 à 10 éléments)-alkyle en C₁-C₆ facultativement substitué, carbocyclyle en C₃-₁₀ facultativement substitué, (carbocyclyl)alkyle en C₄₋₁₀ facultativement substitué, et (hétérocyclyl de 5 à 10 éléments)-alkyle en C₁-C₆ facultativement substitué,
la méthode est effectuée à une température d'au moins au moins 0°C, au moins 10°C, au moins 20°C, au moins 30°C, au moins 40°C, au moins 50°C, au moins 60°C, au moins 70°C, au moins 80°C, au moins 90°C, au moins 100°C, au moins 110°C, au moins 120°C, au moins 130°C, au moins 140°C, au moins 150°C, au moins 160°C, au moins 170°C, ou au moins 180°C.

2. Méthode de fabrication d'un composé de Formule (I), comprenant les étapes consistant à :
(a) faire réagir un composé de Formule (1) et R₁R₂-NH 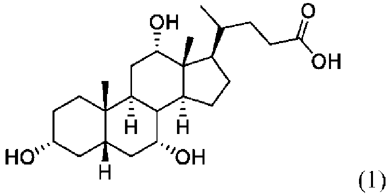 pour former un composé de Formule (2) : 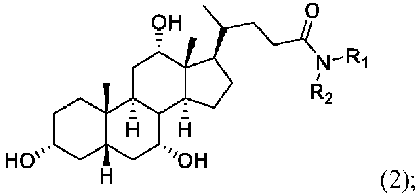
(b) faire réagir un composé de Formule (2) avec un composé de Formule (A), 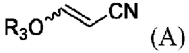 en présence d'acide et d'un catalyseur de transfert de phase, pour former un composé de Formule (3) : 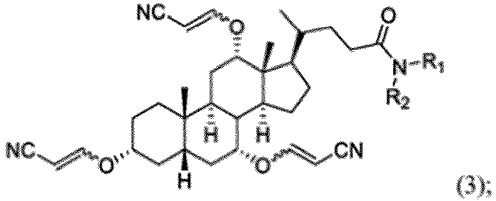 et
(c) soumettre un composé de Formule (3) à des conditions réductrices pour former un composé de Formule (I) : 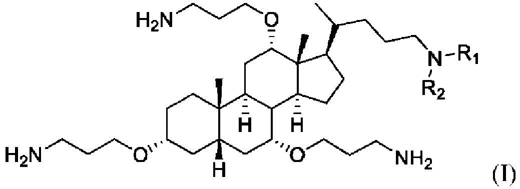 où :
R₁ et R₂ sont indépendamment choisis parmi le groupe constitué d'hydrogène, alkyle en C₁-C₁₄ facultativement substitué, alcényle en C₂-C₂₄ facultativement substitué, alcynyle en C₂-C₂₄ facultativement substitué, aryle en C₆ ou C₁₀ facultativement substitué, hétéroaryle de 5 à 10 éléments facultativement substitué, hétérocyclyle de 5 à 10 éléments facultativement substitué, aralkyle en C₇₋₁₃ facultativement substitué, (hétéroaryle de 5 à 10 éléments)-alkyle en C₁-C₆ facultativement substitué, carbocyclyle en C₃₋₁₀ facultativement substitué, (carbocyclyl)alkyle en C₄₋₁₀ facultativement substitué, (hétérocyclyle de 5 à 10 éléments)-alkyle en C₁-C₆ facultativement substitué, amido facultativement substitué, et un groupe protecteur d'amine approprié ; et
R₃ est choisi parmi le groupe constitué d'alkyle en C₁-C₂₄ facultativement substitué, alcényle en C₂-C₂₄ facultativement substitué, alcynyle en C₂-C₂₄ facultativement substitué, hétérocyclyle de 5 à 10 éléments facultativement substitué, aralkyle en C₇₋₁₃ facultativement substitué, (hétéroaryle de 5 à 10 éléments)-alkyle en C₁-C₆ facultativement substitué, carbocyclyle en C₃-₁₀ facultativement substitué, (carbocyclyl)alkyle en C₄₋₁₀ facultativement substitué, et (hétérocyclyl de 5 à 10 éléments)-alkyle en C₁-C₆ facultativement substitué.

3. Méthode selon la revendication 1 ou 2, dans laquelle R₁ est de l'hydrogène et R₂ est choisi parmi le groupe constitué d'hydrogène, alkyle en C₁-C₂₄ facultativement substitué, alcényle en C₂-C₂₄ facultativement substitué, alcynyle en C₂-C₂₄ facultativement substitué, aryle en C₆ ou C₁₀ facultativement substitué, hétéroaryle de 5 à 10 éléments facultativement substitué, hétérocyclyle de 5 à 10 éléments facultativement substitué, aralkyle en C₇₋₁₃ facultativement substitué, (hétéroaryle de 5 à 10 éléments)-alkyle en C₁-C₆ facultativement substitué, carbocyclyle en C₃₋₁₀ facultativement substitué, (carbocyclyl)alkyle en C₄₋₁₀ facultativement substitué, (hétérocyclyle de 5 à 10 éléments)-alkyle en C₁-C₆ facultativement substitué, et un groupe protecteur d'amine approprié.

4. Méthode selon l'une quelconque des revendications 1 à 3, dans laquelle R₁ est de l'hydrogène et R₂ est choisi parmi le groupe constitué d'alkyle en C₁-C₁₄ facultativement substitué, alcényle en C₂-C₂₄ facultativement substitué, alcynyle en C₂-C₂₄ facultativement substitué, aryle en C₆ ou C₁₀ facultativement substitué, hétéroaryle de 5 à 10 éléments facultativement substitué, hétérocyclyle de 5 à 10 éléments facultativement substitué, aralkyle en C₇₋₁₃ facultativement substitué, (hétéroaryle de 5 à 10 éléments)-alkyle en C₁-C₆ facultativement substitué, carbocyclyle en C₃₋₁₀ facultativement substitué, (carbocyclyl)alkyle en C₄₋₁₀ facultativement substitué, et (hétérocyclyle de 5 à 10 éléments)-alkyle en C₁-C₆ facultativement substitué.

5. Méthode selon l'une quelconque des revendications 1 à 4, dans laquelle R₁ est de l'hydrogène et R₂ est un alkyle en C₁-C₁₄ facultativement substitué.

6. Méthode selon l'une quelconque des revendications 1 à 5, dans laquelle le composé de Formule (I) est choisi parmi le groupe constitué de :

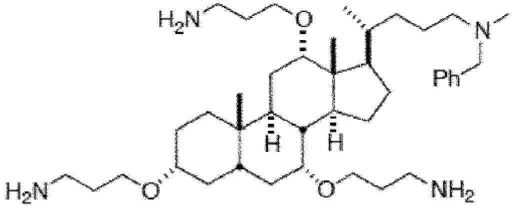

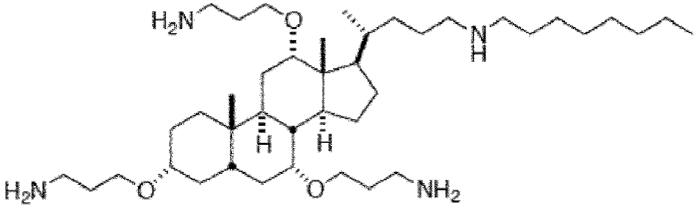

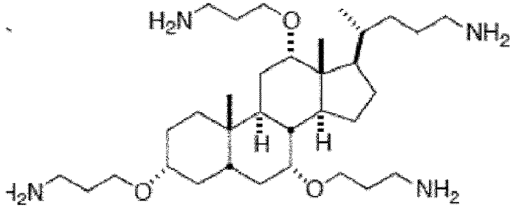

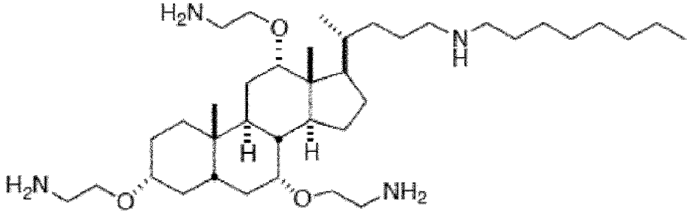

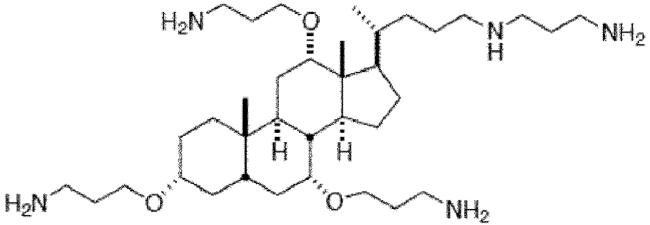

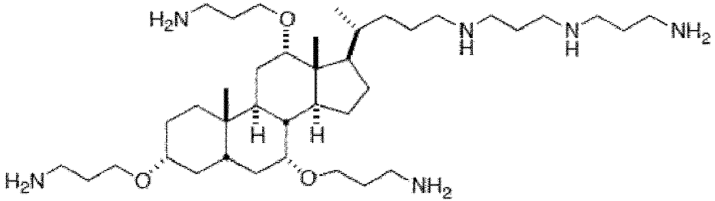

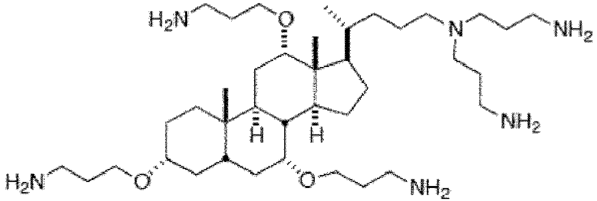

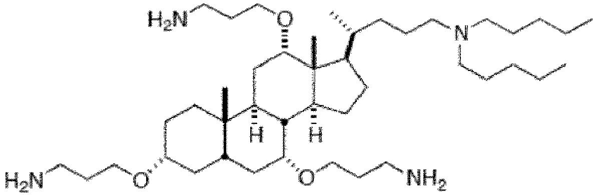

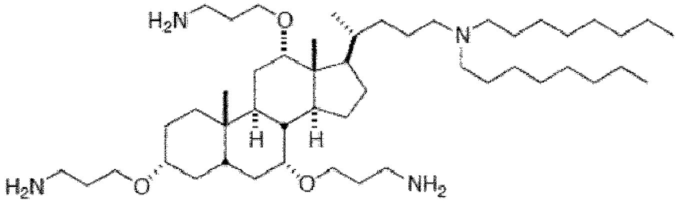

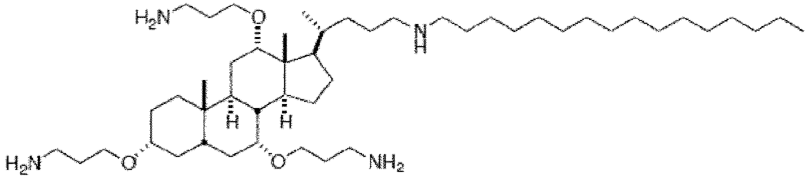

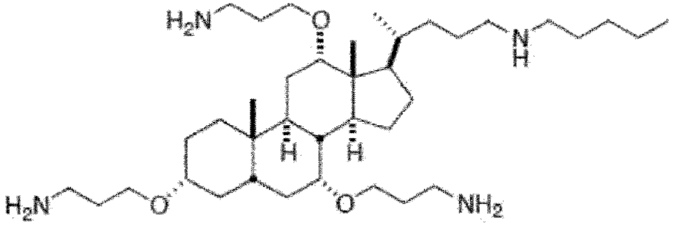

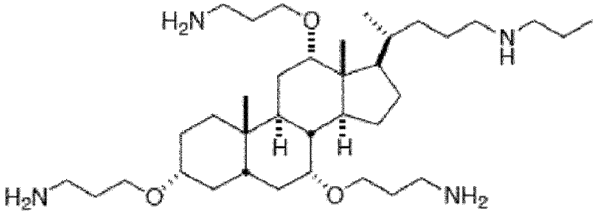

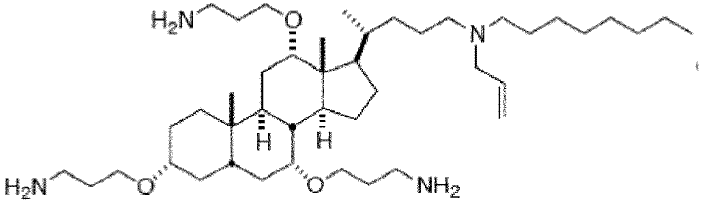

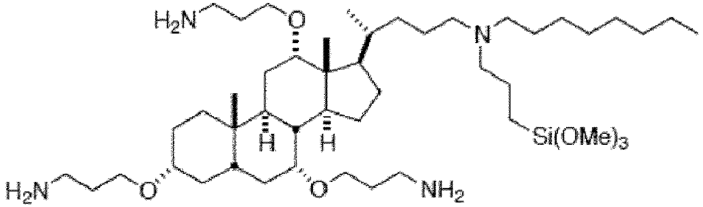

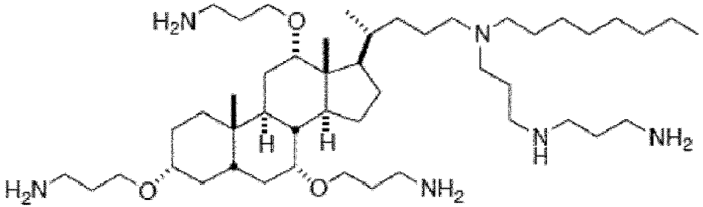

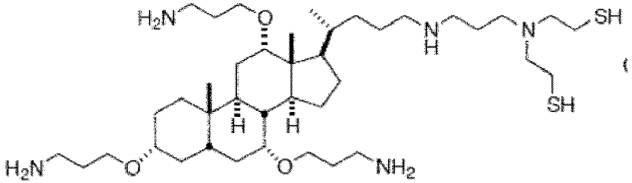

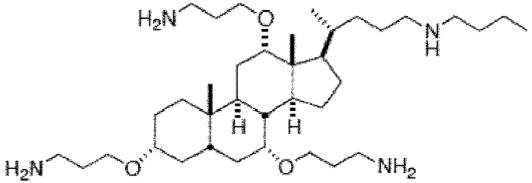

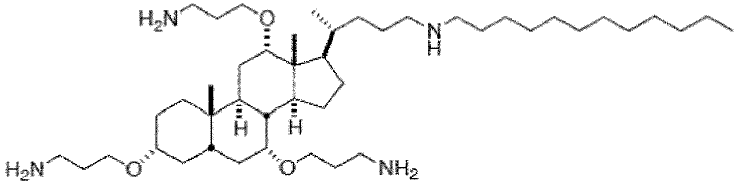

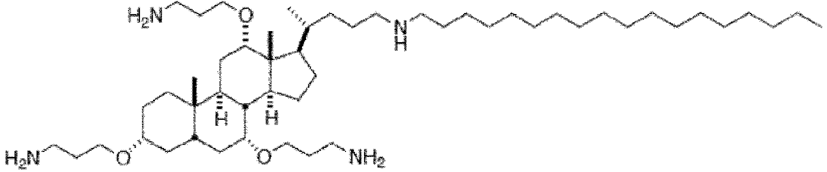

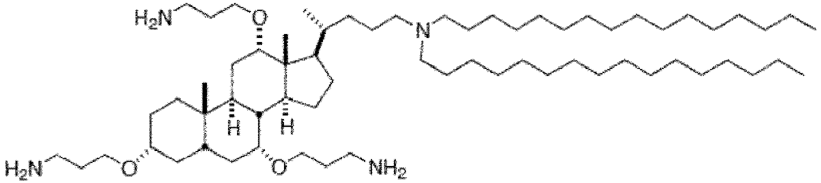

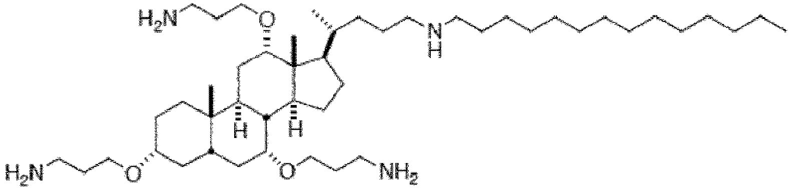

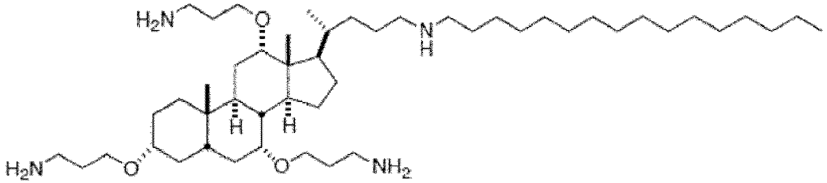

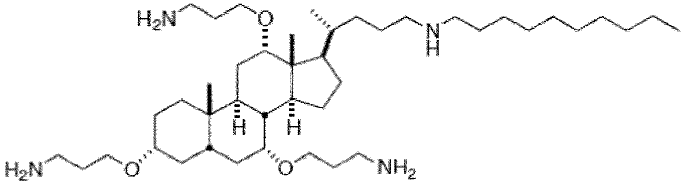

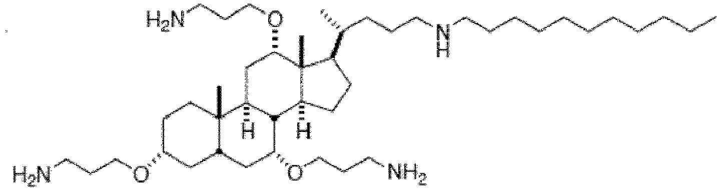

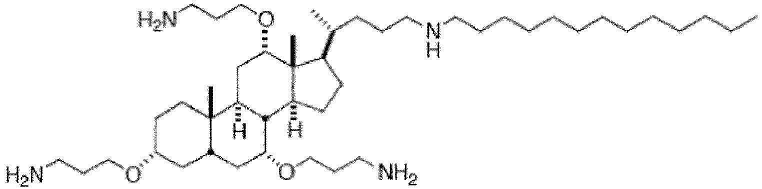

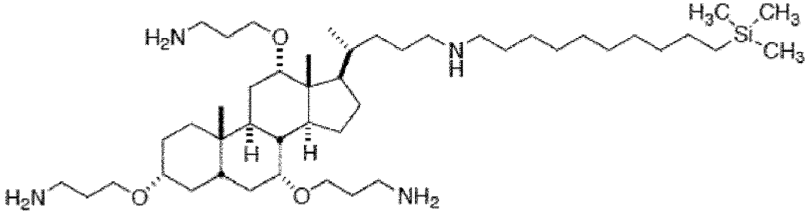

7. Méthode selon la revendication 1, dans laquelle le composé de Formule (II) est choisi parmi le groupe constitué de : 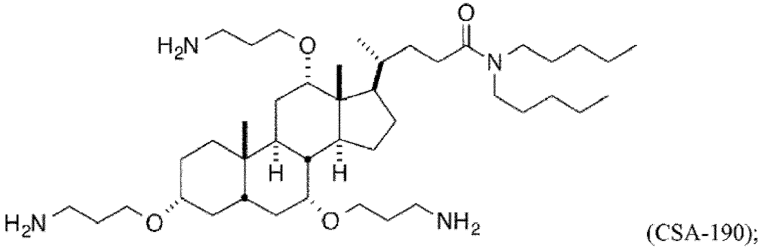 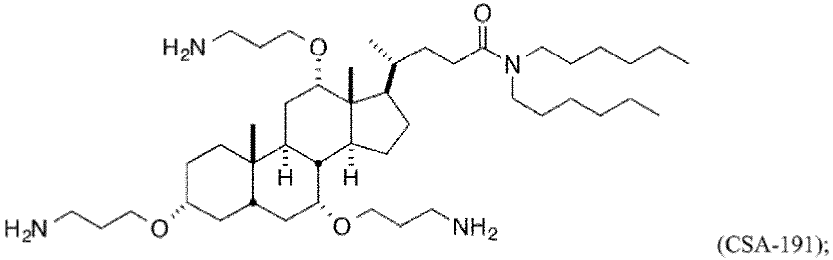 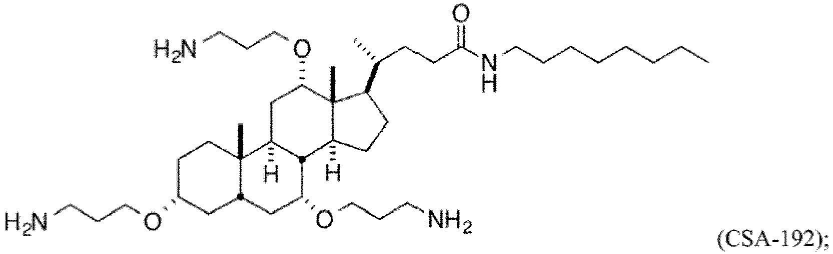 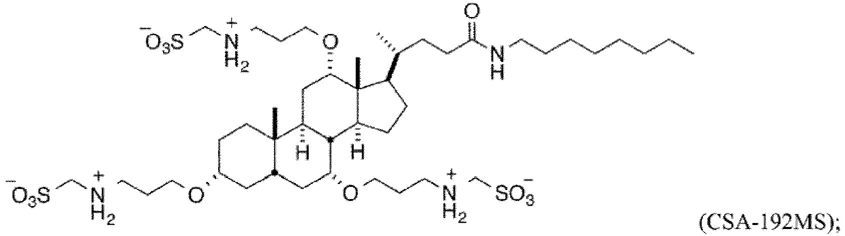 et des sels de ceux-ci.

8. Méthode selon l'une quelconque des revendications 1 à 7, dans laquelle la méthode est effectuée dans un ou plusieurs solvants choisis parmi le groupe constitué d'eau, acide acétique, acide formique, acide chlorhydrique, acide bromhydrique, DMF, DMSO, DCM, NMP, chloroforme, THF, 2-méthyl-THF, benzène, toluène, trifluorotoluène, dioxane, méthanol, éthanol, acétate d'éthyle, acétone, ou toute combinaison de ceux-ci.

9. Méthode selon l'une quelconque des revendications 1 à 8, dans laquelle la méthode est efffectuée en moins de 36 heures, moins de 35 heures, moins de 34 heures, moins de 33 heures, moins de 32 heures, moins de 31 heures, moins de 30 heures, moins de 29 heures, moins de 28 heures, moins de 27 heures, moins de 26 heures, moins de 25 heures, moins de 24 heures, moins de 23 heures, moins de 22 heures, moins de 21 heures, moins de 20 heures, moins de 19 heures, moins de 18 heures, moins de 17 heures, moins de 16 heures, moins de 15 heures, moins de 14 heures, moins de 13 heures, moins de 12 heures, moins de 11 heures, moins de 10 heures, moins de 9 heures, moins de 8 heures, moins de 7 heures, moins de 6 heures, moins de 5 heures, moins de 4 heures, moins de 3 heures, moins de 2 heures ou moins de 1 heure.

10. Méthode selon l'une quelconque des revendications 1 à 9, dans laquelle la méthode fournit au moins un gramme, au moins 10 grammes, et au moins 100 grammes, au moins 500 grammes, au moins un kilogramme, au moins 10 kilogrammes, au moins 50 kilogrammes, au moins 100 kilogrammes, ou au moins 500 kilogrammes, d'un composé de Formule (I) ou d'un composé de Formule (II).

11. Méthode selon l'une quelconque des revendications 1 à 10, dans laquelle la méthode est essentiellement réalisée à pression ambiante.

12. Méthode selon l'une quelconque des revendications 1 à 11, dans laquelle la méthode n'exclut pas délibérément la présence d'oxygène ou est essentiellement réalisée dans une atmosphère ne contenant pas d'oxygène.

13. Méthode selon l'une quelconque des revendications 1 à 12, dans laquelle la méthode est effectuée à une vitesse de mélange d'au moins 1 tr/min, au moins 10 tr/min, au moins 20 tr/min, au moins 30 tr/min, au moins 40 tr/min, au moins 50 tr/min, au moins 60 tr/min, au moins 70 tr/min, au moins 80 tr/min, au moins 90 tr/min, au moins 100 tr/min, au moins 110 tr/min, au moins 120 tr/min, au moins 200 tr/min, ou au moins 500 tr/min.
